(19) **Europäisches Patentamt**
**European Patent Office**
**Office européen des brevets**

(11) **EP 2 635 348 B1**

(12) **EUROPEAN PATENT SPECIFICATION**

(45) Date of publication and mention
of the grant of the patent:
**02.03.2016 Bulletin 2016/09**

(51) Int Cl.:
***A61N 7/00*** *(2006.01)*

(21) Application number: **11781975.5**

(22) Date of filing: **04.11.2011**

(86) International application number:
**PCT/US2011/059342**

(87) International publication number:
**WO 2012/061713 (10.05.2012 Gazette 2012/19)**

(54) **HIGH INTENSITY FOCUSED ULTRASOUND APPARATUSES FOR RENAL NEUROMODULATION**

HOCHENERGIEFOKUSSIERTE ULTRASCHALLVORRICHTUNGEN FÜR DIE RENALE NEUROMODULATION

APPAREILS À ULTRASONS FOCALISÉS DE HAUTE INTENSITÉ POUR LA NEUROMODULATION RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.11.2010 US 940922**

(43) Date of publication of application:
**11.09.2013 Bulletin 2013/37**

(73) Proprietor: **Medtronic Ardian Luxembourg S.à.r.l.**
**2124 Luxembourg (LU)**

(72) Inventors:
• **EMERY, Charles D.**
**Santa Rosa, California 95403 (US)**
• **GELFAND, Mark**
**Santa Rosa, California 95403 (US)**
• **LEVIN, Howard R.**
**Santa Rosa, California 95403 (US)**
• **ZARINS, Denise**
**Santa Rosa, California 95403 (US)**

(74) Representative: **Zimmermann & Partner**
**Patentanwälte mbB**
**Postfach 330 920**
**80069 München (DE)**

(56) References cited:
**WO-A1-2011/046879      WO-A2-2008/003058**
**WO-A2-2011/130531      US-A1- 2010 168 731**

**Description**

TECHNICAL FIELD

The present disclosure relates to high intensity ultrasound apparatuses, systems and methods for intravascular neuromodulation and, more particularly, to high intensity ultrasound apparatuses for application of energy to a renal artery.

BACKGROUND

Hypertension, heart failure, chronic kidney disease, insulin resistance, diabetes and metabolic syndrome represent a significant and growing global health issue. Current therapies for these conditions include non-pharmacological, pharmacological and device-based approaches. Despite this variety of treatment options, the rates of control of blood pressure and the therapeutic efforts to prevent progression of these disease states and their sequelae remain unsatisfactory. Although the reasons for this situation are manifold and include issues of non-compliance with prescribed therapy, heterogeneity in responses both in terms of efficacy and adverse event profile, and others, it is evident that alternative options are required to supplement the current therapeutic treatment regimes for these conditions.

Reduction of sympathetic renal nerve activity (e.g., via denervation), may reverse these processes. Ardian, Inc., of Palo Alto, CA, has discovered that an energy field may initiate renal neuromodulation via denervation caused by irreversible electroporation, electrofusion, apoptosis, necrosis, ablation, thermal alteration, alteration of gene expression, or another suitable modality. WO 2008/003058 describes systems for thermally-induced renal neuromodulation employing ultrasound.

It does not teach the transducers' positionability as set out in claim 1.

SUMMARY

The hollowing summary is provided for the benefit of the reader only, and is not intended to limit the disclosure in any way. The present application provides apparatuses, systems and methods for achieving high intensity focused ultrasound-induced renal neuromodulation (i.e., rendering a nerve inert or inactive or otherwise completely or partially reducing the nerve in function) via intravascular access.

The invention is defined in claim 1.

Embodiments of the present disclosure relate to apparatuses, systems, and methods that incorporate a catheter treatment device having one or more ultrasound transducers. The catheter is associated with an ultrasound transducer configured to deliver ultrasound energy to a renal artery after being inserted via an intravascular path that includes a femoral artery, an iliac artery and the aorta. In particular embodiments, the ultrasound transducer may be positioned within the renal artery or within the abdominal aorta. The ultrasound transducer may be configured to provide treatment energy as well as to provide imaging information, which may facilitate placement of the transducer relative to the renal artery, optimize energy delivery and/or, provide tissue feedback (e.g. determine when treatment is complete). Further, depending on the particular arrangement of the ultrasound transducer, the lesion created by the application of ultrasound energy may be limited to very specific areas (e.g., focal zones or focal points) on the periphery of the artery wall or on the nerves themselves. Indeed, because a transducer may be located within the abdominal aorta but focused on locations in and around the renal artery, blood may flow in and around the focal zones while treatment is applied, which may assist in cooling the interior wall of the artery during the treatment. In such a manner, the lesions may be limited to the exterior surface of the renal artery, which in turn may provide the advantage of more specific targeting of the treatment energy.

BRIEF DESCRIPTION OF THE DRAWINGS

Figure 1 is a conceptual illustration of the sympathetic nervous system (SNS) and how the brain communicated with the body via the SNS.

Figure 2 is an enlarges anatomic view of nerves innervating a left kidney to form the renal plexus surrounding the left renal artery.

Figures 3A and 3B provide anatomic and conceptual views of a human body, respectively, depicting neural efferent and efferent communication between the brain and kidneys.

Figures 4A and 4B are, respectively, anatomic views of the arterial and venous vasculatures of a human.

Figure 5 is an anatomic view of a system for achieving high intensity focused ultrasound renal neuromodulation that includes an external ultrasound energy generator and a treatment device that is inserted within a patient's vascular system.

Figure 6 is a view of a treatment device that includes an inflatable balloon deployed within a renal artery.

Figure 7A is a view of a treatment device that includes a deflectable tip within a renal artery.

Figure 7B is a cross-sectional view of the renal artery with a treatment device of Figure 7A.

Figure 7C is a side view of a distal region of the treatment device of Figure 7A showing a concave focusing cavity.

Figure 7D is a side view of an alternative tip region including a spherical balloon filling the concave focusing cavity.

Figure 7E is a side view of an alternative tip region including a semispherical balloon filling the concave focusing cavity.

Figure 7F is a side view of an alternative distal region including a concave focusing cavity in an orientation orthogonal to the elongated shaft.

Figure 8 is a view of a treatment device including an acoustically conductive expandable balloon within a renal artery.

Figure 9A is a view of a treatment device that includes a convex reflector to focus the ultrasound energy.

Figure 9B is a view of a treatment device that includes a convex reflector to focus the ultrasound energy.

Figure 10 is a view of a treatment device that includes a balloon that acts as an acoustic lens.

Figure 11 is a system-level view of the treatment device of Figure 10.

Figure 12 is a view of a treatment device that includes a toroidal acoustically conductive balloon that acts as an acoustic lens.

Figure 13 is a view of a treatment device including an ultrasound transducer positioned within a renal artery an an ultrasound transducer positioned within an abdominal aorta.

Figure 14A is a view of a treatment device that includes an ultrasound transducer with rotational freedom relative to an axis of a catheter shaft and the ability to be vertically deflected relative to an axis of the catheter shaft.

Figure 14B is a view of a treatment device that includes a transducer with imagine and treatment modalities.

Figure 14C is a view of a treatment device that includes a concave treatment transducer.

Figure 14D is a view of an alternative treatment device that includes a transducer with imaging and treatment modalities.

Figure 14E is a view of an alternative treatment device that includes a transducer with imaging and treatment modalities.

Figure 14F is a view of an alternative treatment device that includes a transducer with adjacent imaging and treatment modalities.

Figure 14G is a view of an alternative treatment device that includes a transducer with adjustable imaging and treatment modalities.

Figure 15 is a cross-sectional view of a treatment device through an aortic transducer.

Figure 16 is a partial side view of the aortic transducer of treatment device showing left and right transducer regions.

Figure 17 is cross-sectional view of the aortic transducer Figure 16 showing left and right transducer regions.

Figure 18 is an example of an energy delivery algorithm that may be used in conjunction with the system of Figure 5.

Figure 19 is a kit for packaging components of the system of Figure 5.

DETAILED DESCRIPTION

Although the disclosure hereof is detailed and exact to enable those skilled in the art to practice the disclosed technologies, the physical embodiments herein disclosed merely exemplify the various aspects of the disclosure, which may be embodied in other specific structures. While the preferred embodiment has been described, the details may be changed without departing from the disclosure, which is defined by the examples.

I. High Intensity Focused Ultrasound for Renal Neuromodulation

In catheter systems for intravascular application of energy to vascular tissue, in order to achieve the therapeutic effect, the energy delivery element is generally placed as close to the tissue to be treated as possible. However, since highest energy density is closest to the tip of the catheter, the greatest tissue effect drops off from the tip. For intravascular renal neuromodulation applications, this may result in higher energy delivery to the interior of the renal artery with less energy delivered to the nerves themselves. As such, achieving suitable energy delivery that modulates the nerves without overheating the renal artery is complex.

In cardiovascular ablation technologies, deep scarring of heart muscle, known as transmural lesions, are created to control arrhythmias. The goal of renal denervation differs from cardiac ablation in that creation of transmural lesions in the blood vessel walls is generally not desired. Nerves are more fragile than cardiac tissue and stop conducting signals when heated but not necessarily scarred. At the same time, nerves are located some distance away from the blood vessel wall where the heating instrument may be applied. This creates a need for better and improved denervation methods and devices. However, with certain energy modalities, it may be technically challenging to create segmented or continuous circumferential linear lesions desired for renal neuromodulation. This results in time consuming ablation procedures, increasing the discomfort and risk for complications for both, the patient and the physician.

Provided herein are catheter apparatuses, methods, and systems that incorporate high intensity focused ultrasound (HIFU) ultrasound as an energy source to therapeutically treat tissues. Mechanical vibrations above the threshold of the human hearing are called ultrasound. Ultrasound waves may propagate through living tissue and fluids without causing any harm to the cells. By focusing highly energetic ultrasound waves to a well defined volume, local heat rise (e.g. >56°C and typically up to 80°C) occurs and causes rapid tissue necrosis by coagulative necrosis. Fortunately, a steep temperature gradient is observed between the focus and the surrounding tissue allowing for the production of sharply demarcated lesions and reducing collateral damage. The controller degree of heating and damage may be achieved by dosing of energy (electric power delivered to the source and the duration of application). Pulsed ultrasound may be also used to control tissue modification. Furthermore, frequency selection may be used to control tissue modification.

Another mechanism by which HIFU destroys tissue is called acoustic cavitation. This process is based on vibration of cellular structures causing local hyperthermia and mechanical stress by bubble formation due to rapid changes in local pressure leading to cell death. It is appreciated that for the purpose of this disclosure, necrosis of tissue may not be needed. Nerves are more fragile than the surrounding tissue and may be effectively functionally disabled by heating to a temperature that does not cause necrosis. In particular embodiments, the heating of selected tissue with ultrasonic waves to a temperature above normal range may be referred to as "sonication."

HIFU presents several advantages over other energy modalities for renal denervation. In particular embodiments, ultrasound is capable of focusing energy on one or more focal points some distance from the source of ultrasonic waves. As opposed to energy application from a thermal or radiofrequency source (e.g., RF ablation) that distributes energy locally at the point of application, HIFU may focus energy at a distant point with targeted focusing of the ultrasound radiation. As such, in HIFU, an energy source may be remote (e.g., not within the renal artery) to achieve energy application and renal neuromodulation without disturbing tissue located proximally or distally from the intended treatment zone. Targeted energy delivery may be achieved without precise placement of a catheter device, which may allow greater operator flexibility and may provide additional benefit to patients whose anatomy may make placement of catheter within a renal artery particularly challenging.

In particular embodiments, HIFU emitters may be configured to focus energy on the deep tissue zones one to three millimeters away from the emitter, therefore sparing the intima and media of the renal artery and destroying nerves that may be dispersed between the adventitia and over some distance from the arterial wall. Histological studies show that renal nerves form a plexus of many fibers surrounding the external wall of the renal artery. While some may be embedded in the exterior of the arterial wall, some may be located several millimeters outside. In addition, HIFU may achieve deep tissue heating that may result in more complete destruction of renal nerves. Further, because HIFU techniques may target the nerves while sparing the arterial wall, higher levels of concentrated heat may be applied to the target, thus shortening the procedure. Furthermore, a HIFU device can focus energy at multiple focal points simultaneously which may further reduce procedure time. More thorough destruction of renal nerves with heat may also reduce the chance of nerves re-growing later and the need for repeated procedure.

Embodiments provided herein include a renal artery catheter, for example steerable by the operator, and an acoustic frequency generator. In particular embodiments provided herein, a HIFU catheter may be used in conjunction with a sonic crystal or an array of crystals. In such arrangements, focusing of acoustic energy emitted by the crystal may be achieved with a focusing lens such as a concave cavity. The actual geometry of the cavity determines the distance from the catheter application point to the point of energy focus. To improve safety, the catheter may be equipped with a temperature sensor and temperature control circuits to prevent overheating of tissue and device itself.

In addiction, it may be possible to place a therapeutic transducer in the artery and avoid significant heating of the intima and or media. This ability to remotely treat tissue (i.e., with energy applied via a transducer not in direct contact with the treated tissue) is based on energy concentration of the acoustic focus. Since most of the tissue is thermally insulating, the heating that occurs due to the acoustic concentration is not quickly conducted away from the focus. The frequency chosen for HIFU is a function of the expected attenuation, the containment of the beam both laterally and axially, and the treatment depths. It particular embodiments, frequencies ranging from below 1 MHz for deep depths to over 5 MHz for shallow depths may be used in conjunction with the embodiments provided herein. However, it should be noted that these ranges are not meant to be limiting and other frequencies may provide a therapeutic effect.

In addition, ultrasound has also been used expensively to image the soft tissues of the body and, in certain embodiments, the imaging capabilities of ultrasound techniques may be used for device placement and targeting. In this manner, a HIFU device for neuromodulation may be used for imaging the renal artery, targeting the renal nerves, determining the optimal treatment power or dose, and/or determining when to halt a treatment. In particular embodiments, the disclosed embodiments utilize therapeutic ultrasound and/or diagnostic ultrasound for successful renal denervation.

II. <u>Pertinent Anatomy and Physiology</u>

A. <u>The Sympathetic Nervous System</u>

The Sympathetic Nervous System (SNS) is a branch of the autonomic nervous system along with the enteric nervous system and parasympathetic nervous system. It is always active at a basal level (called sympathetic tone) and becomes more active during times of stress. Like other parts of the nervous system, the sympathetic nervous system operates through a series of interconnected neurons. Sympathetic neurons are frequently considered part of the peripheral nervous system (PNS), although many lie within the central nervous system (CNS). Sympathetic neurons of the spinal cord (which is part of the CNS) communicate with peripheral sympathetic neurons via a series of sympathetic ganglia. Within the ganglia, spinal cord sympathetic neurons join peripheral sympathetic neurons through synapses. Spinal cord sympathetic neurons are therefore called presynaptic (or preganglionic) neurons, while peripheral sympathetic neurons are called postsynaptic (or postganglionic) neurons.

At synapses within the sympathetic ganglia, preganglionic sympathetic neurons release acetylcholine, a chemical messenger that binds and activates nicotinic acetylcholine receptors on postganglionic neurons. In response to this stimulus, postganglionic neurons principally release noradrenaline (norepinephrine). Prolonged activation may elicit the release of adrenaline from the adrenal medulla.

Once released, norepinephrine and epinephrine bind adrenergic receptors on peripheral tissues. Binding to adrenergic receptors causes a neuronal and hormonal response. The physiologic manifestations include pupil dilation, increased heart rate, occasional vomiting, and increased blood pressure. Increased sweating is also seen due to binding of cholinergic receptors of the sweat glands.

The sympathetic nervous system is responsible for up- and down-regulating many homeostatic mechanisms in living organisms. Fibers from the SNS innervate tissues in almost every organ system, providing at least some regulatory function to things as diverse as pupil diameter, gut motility, and urinary output. This response is also known as *sympatho-adrenal response* of the body, as the preganglionic sympathetic fibers that end in the adrenal medulla (but also all other sympathetic fibers) secrete acetylcholine, which activates the secretion of adrenaline (epinephrine) and to a lesser extent noradrenaline (norepinephrine). Therefore, this response that acts primarily on the cardiovascular system is mediated directly via impulses transmitted through the sympathetic nervous system and indirectly via catecholamines secreted from the adrenal medulla.

Science typically looks at the SNS as an automatic regulation system, that is, one that operates without the intervention of conscious thought. Some evolutionary theorists suggest that the sympathetic nervous system operated in early organisms to maintain survival as the sympathetic nervous system is responsible for priming the body for action.

One example of this priming is in the moments before waking, in which sympathetic outflow spontaneously increases in preparation for action.

1. The Sympathetic Chain

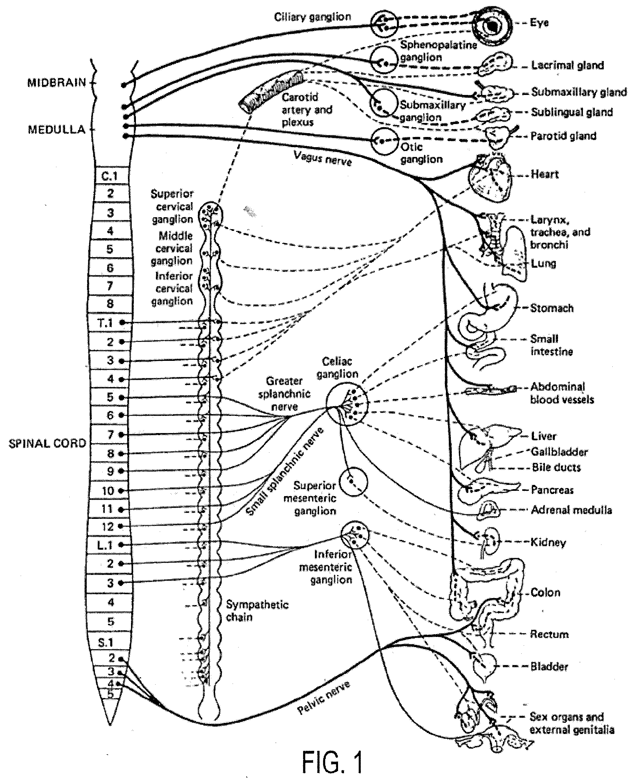

As shown in Figure 1, the SNS provides a network of nerves that allows the brain to communicate with the body. Sympathetic nerves originate inside the vertebral column, toward the middle of the spinal cord in the intermediolateral cell column (or lateral horn), beginning at the first thoracic segment of the spinal cord and are thought to extend to the second or third lumbar segments. Because its cells begin in the thoracic and lumbar regions of the spinal cord, the SNS is said to have a *thoracolumbar outflow.* Axons of these nerves leave the spinal cord through the anterior rootlet/root. They pass near the spinal (sensory) ganglion, where they enter the anterior rami of the spinal nerves. However, unlike somatic innervation, they quickly separate out through white rami connectors which connect to either the paravertebral (which lie near the vertebral column) or prevertebral (which lie near the aortic bifurcation) ganglia expending alongside the spinal column.

In order to reach the target organs and glands, the axons should travel long distanced in the body, and, to accomplish this, many axons relay their message to a second cell through synaptic transmission. The ends of the axons link across a space, the synapse, to the dendrites of the second cell. The first cell (the presynaptic cell) sends a neurotransmitter across the synaptic cleft where it activates the second cell (the postsynaptic cell). The message is then carried to the final destination.

In the SNS and other components of the peripheral nervous system, these synopses are made at sites called ganglia. The cell that sends its fiber is called a preganglionic cell, while the cell whose fiber leaves the ganglion is called a postganglionic cell. As mentioned previously, the preganglionic cells of the SNS are located between the first thoracic (T1) segment and third lumbar (L3) segments of the spinal cord. Postganglionic cells have their cell bodies in the ganglia and send their axons to target organs or glands.

The ganglia include not just the sympathetic trunks but also the cervical ganglia (superior, middle and inferior), which sends sympathetic nerve fibers to the head and thorax organs, and the celiac and mesenteric ganglia (which send sympathetic fibers to the gut).

2. Innervation of the Kidneys

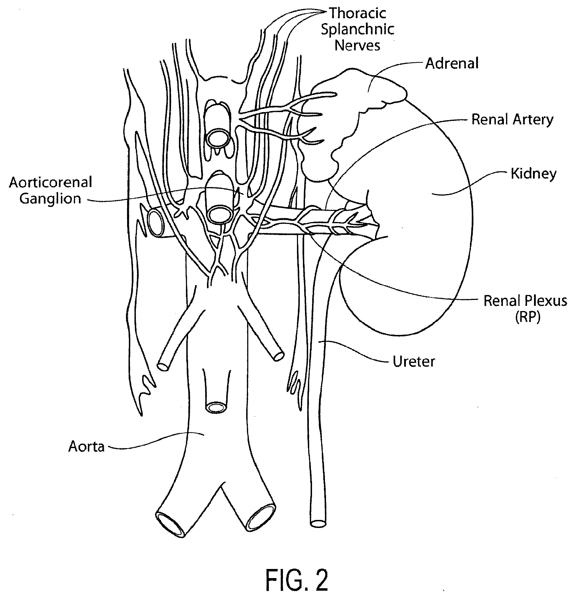

As Figure 2 shows, the kidney is innervated by the renal plexus (RP), which is intimately associated with the renal artery. The renal plexus is an autonomic plexus that surround the renal artery and is embedded within the adventitia of the renal artery. The renal plexus extends along the renal artery until it arrives at the substance of the kidney. Fibers contributing to the renal plexus arise from the celiac ganglion, the superior mesenteric ganglion, the aorticorenal ganglion and the aortic plexus. The renal plexus (RP), also referred to as the renal nerve, is predominantly comprised of sympathetic components. There is no (or at least very minimal) parasympathetic innervation of the kidney.

Preganglionic neuronal cell bodies are located in the intermediolateral cell column of the spinal cord. Preganglionic axons pass through the paravertebral ganglia (they do not synapse) to become the lesser splanchnic nerve, the least splanchnic nerve, first lumbar splanchnic nerve, second lumbar splanchnic nerve, and travel to the celiac ganglion, the superior mesenteric ganglion, and the aorticorenal ganglion. Postganglionic neuronal cell bodies exit the celiac ganglion, the superior mesenteric ganglion, and the aorticorenal ganglion to the renal plexus (RP) and are distributed to the renal vasculature.

3. Renal Sympathetic Neural Activity

Messages travel through the SNS in a bidirectional flow. Efferent messages may trigger changes in different parts of the body simultaneously. For example, the sympathetic nervous system may accelerate heart rate; widen bronchial passages; decrease motility (movement) of the large intestine; construct blood vessels; increase peristalsis in the esophagus; cause pupil dilation, piloerection (goose bumps) and perspiration (sweating); and raise blood pressure. Efferent messages carry signals from various organs and sensory receptors in the body to other organs and, particularly, the brain.

Hypertension, heart failure and chronic kidney disease are a few of many disease states that result from chronic activation of the SNS, especially the renal sympathetic nervous system. Chronic activation of the SNS is a maladaptive response that drives the progression of these disease states. Pharmaceutical management of the renin-angiotensin-aldosterone system (RAAS) has been a longstanding, but somewhat ineffective, approach for reducing over-activity of the SNS.

As mentioned above, the renal sympathetic nervous system has been identifies as a major contributor to the complex pathophysiology of hypertension, states of volume overload (such as heart failure), and progressive renal disease, both experimentally and in humans. Studies employing radiotracer dilution methodology to measure overflow of norepinephrine from the kidneys to plasma revealed increased renal norepinephrine (NE) spillover rates in patients with essential hypertension, particularly so in young hypertensive subjects, which in concert with increased NE spillover from the heart, is consistent with the hemodynamic profile typically seen in early hypertension and characterized by an increased heart rate, cardiac output and renovascular resistance. It is now known that essential hypertension is commonly neurogenic, often accompanied by pronounced sympathetic nervous system overactivity.

Activation of cardiorenal sympathetic nerve activity is even more pronounced in heart failure, as demonstrated by an exaggerated increase of NE overflow from the heart and the kidneys to plasma in this patient group. In line with this notion is the recent demonstration of a strong negative predictive value of renal sympathetic activation on all-cause mortality and heart transplantation in patients with congestive heart failure, which is independent of overall sympathetic activity, glomerular filtration rate and left ventricular ejection fraction. These findings support the notion that treatment regimens that are designed to reduce renal sympathetic stimulation have the potential to improve survival in patients with heart failure.

Both chronic and end stage renal disease are characterized by heightened sympathetic nervous activation. In patients with end stage renal disease, plasma levels of norepinephrine above the median have been demonstrated to be predictive for both all cause death and death from cardiovascular disease. This is also true for patients suffering from diabetic or contrast nephropathy. There is compelling evidence that suggests that sensory efferent signals originating from the diseased kidneys are major contributors to the initiation and sustainment of elevated central sympathetic outflow in this patient group, which facilitates the occurrence of the well know adverse consequences of chronic sympathetic overactivity such as hypertension, left ventricular hypertrophy, ventricular arrhythmias, sudden cardiac death, insulin resistance, diabetes and metabolic syndrome.

(i) Renal Sympathetic Efferent Activity

Sympathetic nerves to the kidneys terminate in the blood vessels, the juxtaglomerular apparatus and the renal tubules. Stimulation of the renal sympathetic nerves causes increased renin release, increased sodium (Na+) reabsorption and a reduction of renal blood flow. These components of the neural regulation of renal function are considerably stimulated in disease states characterized by heightened sympathetic tone and clearly contribute to the rise in blood pressure in hypertensive patients. The reduction of renal blood flow and glomerular filtration rate as a result of renal sympathetic efferent stimulation is likely a cornerstone of the loss of renal function in cardio-renal syndrome, which is renal dysfunction as a progressive complication of chronic heart failure, with a clinical course that typically fluctuates with the patient's clinical status and treatment. Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release) and diuretics (intended to counter the renal sympathetic mediated sodium and water retention). However, the current pharmacologic strategies have significant limitations including limited efficacy, compliance issues, side effects and others.

(ii) Renal Sensory Afferent Nerves Activity

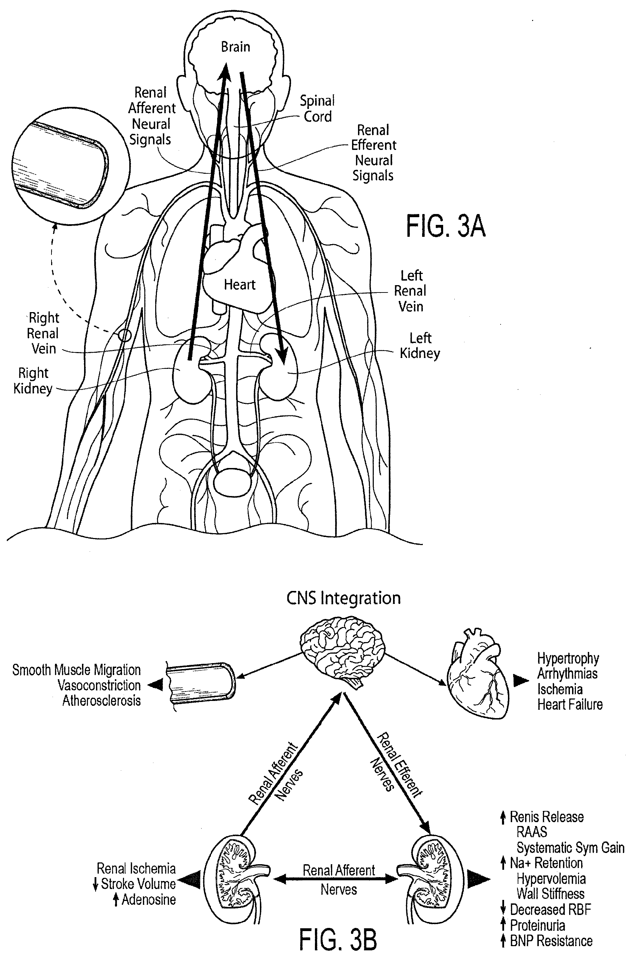

The kidneys communicate with integral structures in the central nervous system via renal sensory afferent nerves. Several forms of "renal injury" may induce activation of sensory afferent signals. For example, renal ischemia, reduction in stroke volume or renal blood flow, or an abundance of adenosine enzyme may trigger activation of afferent neural communication. As shown in Figures 3A and 3B, this afferent communication might be from the kidney to the brain or might be from one kidney to the other kidney (via the central nervous system). These afferent signals are centrally integrated and may result in increased sympathetic outflow. This sympathetic drive is directed towards the kidneys, thereby activating the RAAS and inducting increased renin secretion, sodium retention, volume retention and vasoconstriction. Central sympathetic overactivity also impacts other origans and bodily structures innervated by sympathetic nerves such as the heart and the peripheral vasculature, resulting in the described adverse effects of sympathetic activation, several aspects of which also contribute to the rise in blood pressure.

The physiology therefore suggests that (i) denervation of tissue with efferent sympathetic nerves will reduce inappropriate renin release, salt retention, and reduction of renal blood flow, and that (ii) denervation of tissue with efferent sensory nerves will reduce the systemic contribution to hypertension, and other disease states associated with increased central sympathetic tone, through its direct effect on the posterior hypothalamus as well as the contralateral kidney. In addition to the central hypotensive effects of afferent renal denervation, a desirable reduction of central sympathetic outflow to various other sympathetically innervated organs such as the heart and the vasculature is anticipated.

B. Additional Clinical Benefits of Rental Denervation

As provided above, renal denervation is likely to be valuable in the treatment of several clinical conditions characterized by increased overall and particularly renal sympathetic activity such as hypertension, metabolic syndrome, insulin resistance, diabetes, left ventricular hypertrophy, chronic and end stage renal disease, inappropriate fluid retention in heart failure, cardio-renal syndrome and sudden death. Since the reduction of efferent neural signals contributes to the systemic reduction of sympathetic tone/drive, renal denervation might also be useful in treating other conditions associated with systemic sympathetic hyperactivity. Accordingly, renal denervation may also benefit other origans and bodily structures innervated by sympathetic nerves, including those identifies in Figure 1. For example, a reduction in central sympathetic drive may reduce the insulin resistance that afflicts people with metabolic syndrome and Type II diabetics. Additionally, patients with osteoporosis are also sympathetically activated and might also benefit from the downregulation of sympathetic drive that accompanies renal denervation.

C. Achieving Intravascular Access to the Renal Artery

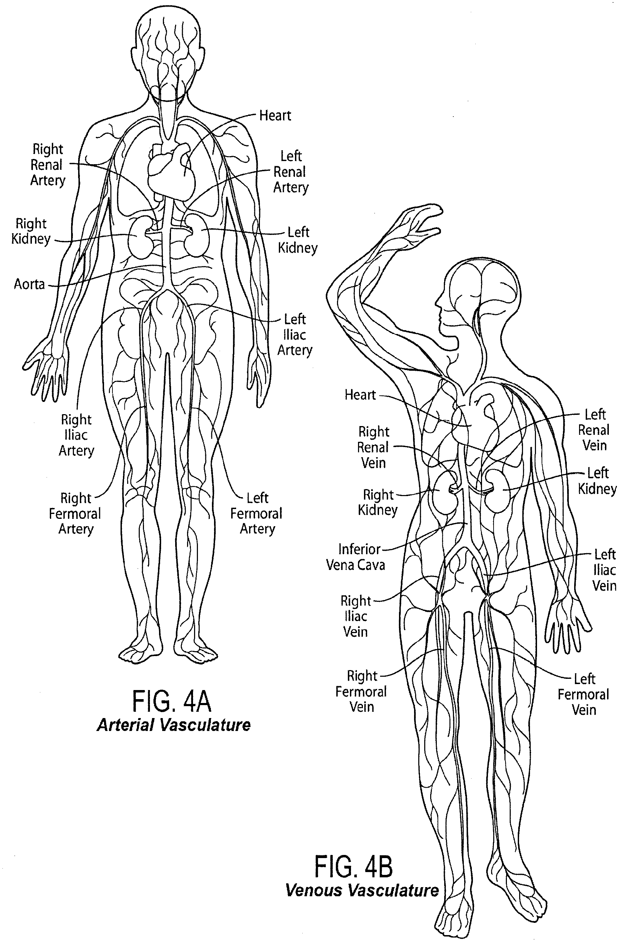

In accordance with the present disclosure, neuromodulation of a left and/or right renal plexus (RP), which is intimately associated with a left and/or right renal artery, may be achieved through intravascular access. As Figure 4A shows, blood moved by contractions of the heart is conveyed from the left ventricle of the heart by the aorta. The aorta descends through the thorax and branches into the left and right renal arteries. Below the renal arteries, the aorta bifurcates at the left and right iliac arteries. The left and right iliac arteries descend, respectively, through the left and right legs and join the left and right femoral arteries.

As Figure 4B shows, the blood collects in veins and returns to the heart, through the femoral veins into the iliac veins and into the inferior vena cava. The inferior vena cava branches into the left and right renal veins. Above the renal veins, the interior vena cava ascends to convey blood into the right atrium of the heart. From the right atrium, the blood is pumped through the right ventricle into the lungs, where it is oxygenated. From the lungs, the oxygenated blood is conveyed into the left atrium. From the left atrium, the oxygenated blood is conveyed by the left ventricle back to the aorta.

As will be described in greater detail later, the femoral artery may be accessed and cannulated at the base of the femoral triangle, just interior to the midpoint of the inguinal ligament. A catheter may be inserted through this access site, percutaneously into the femoral artery and passed into the iliac artery and aorta, into either the left or right rental artery. This comprises an intravascular path that offers minimally invasive access to a respective renal artery and/or other renal blood vessels.

The wrist, upper arm, and shoulder region provide other locations for introduction of catheters into the arterial system. Catheterization of either the radial, brachial, or axillary artery may be utilized in select cases. Catheters introduced via these access points may be passed through the subclavian artery on the left side (or via the subclavian and brachiocephalic arteries on the right side), through the aortic arch, down the descending aorta and into the renal arteries using standard angiographic technique.

D. Properties and Characteristics of the Renal Vasculature

Since neuromodulation of a left and/or right renal plexus (RP) may be achieved in accordance with the present disclosure through intravascular access, properties and characteristics of the renal vasculature may impose constraints upon and/or inform the design of apparatus, systems and methods for achieving such renal neuromodulation. Some of these properties and characteristics may vary across the patient population and/or within a specific patient across time, as well as in response to disease states, such as hypertension, chronic kidney disease, vascular disease, end-stage renal disease, insulin resistance, diabetes, metabolic syndrome, etc. These properties and characteristics, as explained below, may have bearing on the clinical safety and efficacy of the procedure and the specific design of the intravascular device. Properties of interest may include, for example, material/mechanical, spatial, fluid dynamic/hemodynamic and/or thermodynamic properties.

As discussed previously, a catheter may be advanced percutaneously into either the left or right renal artery via a minimally invasive intravascular path. However, minimally invasive renal arterial access may be challenging, for example, because, as compared to some other arteries that are routinely accessed using catheters, the renal arteries are often extremely tortuous, may be of relatively small diameter and/or may be of relatively short length. Furthermore, renal arterial atherosclerosis is common in many patients, particularly those with cardiovascular disease. Renal arterial anatomy also may vary significant from patient to patient, further complicating minimally invasive access. Significant inter-patient variation may be seen, for example, in relative tortuosity, diameter, length and/or atherosclerotic plaque burden, as well as in the take-off angle at which a renal artery branches from the aorta. Apparatus, systems and methods for achieving renal neuromodulation via intravascular access should account for these and other aspects of renal arterial anatomy and its variation across the patient population when minimally invasively accessing a renal artery.

In addition to complicating renal arterial access, specifics of the renal anatomy also complicate establishment of stable contact between neuromodulatory apparatus and a luminal surface or wall of a renal artery. When the neuromodulatory apparatus includes an ultrasound transducer, consistent positioning and contact force application between the ultrasound transducer and the vessel wall may be related to treatment success. In other embodiments, the positioning of the transducer/s relative to a renal artery or abdominal aorta may be considered. However, navigation is impeded by the tight space within a renal artery, as well as tortuosity of the artery. Furthermore, patient movement, respiration and/or the cardiac cycle may cause significant movement of the renal artery relative to the aorta, and the cardiac cycle may transiently distend the renal artery (i.e. cause the wall of the artery to pulse), further complicating establishment of stable contact.

Even after accessing a renal artery and facilitating stable positioning of the neuromodulatory apparatus relative to the artery, nerves in and around the adventia of the artery should be safely modulated via the neuromodulatory apparatus. Safely applying thermal treatment (e.g., sonication) from near or within a renal artery is non-trivial given the potential clinical complications associated with such treatment. For example, the intima and media of the renal artery are highly vulnerable to thermal injury. As discussed in greater detail below, the intima-media thickness separating the vessel lumen from its adventitia means that target renal nerves may be multiple millimeters distant from the luminal surface of the artery. Sufficient energy should be delivered to the target renal nerves to modulate the target renal nerves without excessively heating and desiccating the vessel wall. Another potential clinical complication associated with excessive heating is thrombus formation from coagulating blood flowing through the artery. Given that this thrombus may cause a kidney infarct, thereby causing irreversible damage to the kidney, thermal treatment from within the renal artery should be applied carefully. Accordingly, the complex fluid mechanic and thermodynamic conditions present in the renal artery during treatment, particularly those that may impact heat transfer dynamics at the treatment site, may be important in applying energy, e.g., thermal energy, from within the renal artery.

The neuromodulatory apparatus should also be configured to allow for adjustable positioning and repositioning of the ultrasound transducer proximate to or within the renal artery since location of treatment may also impact clinical safety and efficacy. For example, it may be tempting to apply a full circumferential treatment from within the renal artery given that the rental nerves may be spaced circumferentially around a rental artery. However, the full-circle lesion likely resulting from a continuous circumferential treatment may create a heightened risk of renal artery stenosis, thereby negating any potential therapeutic benefit of the renal neuromodulation. Therefore, the formation of more complex lesions along a longitudinal dimension of the renal artery and/or repositioning of the neuromodulatory apparatus to multiple treatment locations may be desirable. It should be noted, however, that a benefit of creating a circumferential ablation may outweigh the risk of renal artery stenosis or the risk may be mitigated with certain embodiments, or in certain patients and creating a circumferential ablation could be a goal. Additionally, variable positioning and repositioning of the neuromodulatory apparatus may prove to be useful in circumstances where the renal artery is particularly tortuous or where there are proximal branch vessels off the renal artery main vessel, making treatment in certain locations challenging. Manipulation of a device in a renal artery should also consider mechanical injury imposed by the device on the renal artery. Motion of a device in an artery, for example by inserting, manipulating, negotiating bends and so forth, may cause mechanical injury such as dissection, perforation, denuding intima, or disrupting the interior elastic lamina.

Blood flow through a renal artery may be temporarily occluded for a short time with minimal or no complications. However, occlusion for a significant amount of time may cause injury to the kidney such as ischemia. It could be beneficial to avoid occlusion all together or, if occlusion is beneficial to the embodiment, to limit the duration of occlusion, for example to no more than about 3 or 4 minutes.

Based on the above described challenges of (1) renal artery intervention, (2) consistent and stable placement of the treatment element against the vessel wall, (3) safe application of treatment across the vessel wall, (4) positioning and potentially repositioning the treatment apparatus to allow for multiple treatment locations, and (5) avoiding or limiting duration of blood flow occlusion, various independent and dependent properties of the rental vasculature that may be of interest include, for example, vessel diameter, length, intima-media thickness, coefficient of friction and tortuosity; distensibility, stiffness and modulus of elasticity of the vessel wall; peak systolic and end-diastolic blood flow velocity, as well as the mean systolic-diastolic peak blood flow velocity, mean/max volumetric blood flow rate; specific heat capacity of blood and/or of the vessel wall, thermal conductivity of blood and/or of the vessel wall, thermal convectivity of blood flow past a vessel wall treatment site and/or radiative heat transfer; and renal artery motion relative to the aorta, induced by respiration, patient movement, and/or blood flow pulsatility, as well as the take-off angle of a renal artery relative to the aorta. These properties will be discussed in greater detail with respect to the renal arteries. However, dependant on the apparatus, systems and methods utilized to achieve renal neuromodulation, such properties of the renal arteries also may guide and/or constrain design characteristics.

An apparatus positioned within a renal artery should conform to the geometry of the artery. Renal artery vessel diameter, $D_{RA}$, typically is in a range of about 2-10mm, with an average of about 6mm. Renal artery vessel length, $L_{RA}$, between its ostium at the aorta/renal artery juncture and its distal branchings, generally is in a range of about 5-70mm, more generally in a range of about 20-50mm. Since the target renal plexus is embedded within the adventitia of the rental artery, the composite Intima-Media Thickness, IMT, (i.e., the radial outward distance from the artery's luminal surface to the adventitia containing target neural structures) also is notable and generally is in a range of about 0.5-2.5mm, with an average of about 1.5mm. Although a certain depth of treatment is important to reach the target neural fibers, the treatment should not be too deep (e.g., > 5mm from inner wall of the renal artery) to avoid non-target tissue and anatomical structures such as the regnal vein.

Apparatus navigates within a renal artery also should contend with friction and tortuosity. The coefficient of friction, $\mu$, (e.g., static or kinetic friction) at the wall of a renal artery generally is quite low, for example, generally is less than about 0.05, or less than about 0.03. Tortuosity, $\tau$, a measure of the relative twistiness of a curved segment, has been quantified in various ways. The arc-chord ratio defines tortuosity as the length of a curve, $L_{curve}$, divided by the chord, $C_{curve}$, connecting the ends of the curve (i.e., the linear distance separating the ends of the curve):

$$\tau = L_{curve}/C_{curve} \qquad (1)$$

Renal artery tortuosity, as defined by the arc-chord ratio, is generally in the range of about 1-2.

The pressure change between diastole and systole charges the luminal diameter of the rental artery, providing information on the bulk material properties of the vessel. The Distensibility Coefficient, DC, a property dependent on actual blood pressure, captures the relationship between pulse pressure and diameter change:

$$DC = 2*((D_{sys} - D_{dia})/D_{dia})/\Delta P = 2*(\Delta D/D_{dia})/\Delta P, \qquad (2)$$

where $D_{sys}$ is the systolic diameter of the renal artery, $D_{dia}$ is the diastolic diameter of the renal artery, and $\Delta D$ (which generally is less than about 1mm, e.g., in the range of about 0.1mm to 1mm) is the difference between the two diameters:

$$\Delta D = D_{sys} - D_{dia} \qquad (3)$$

The renal arterial Distensibility Coefficient is generally in the range of about 20-50 $kPa^{-1}*10^{-3}$.

The luminal diameter change during the cardiac cycle also may be used to determine renal arterial Stiffness, $\beta$. Unlike the Distensibility Coefficient, Stiffness is a dimensionless property and is independent of actual blood pressure in normotensive patients:

$$\beta = (\ln[BP_{sys}/BP_{dia}])/(\Delta D/D_{dia}) \qquad (4)$$

Renal arterial Stiffness generally is in the range of about 3.5-4.5.

In combination with other geometric properties of the renal artery, the Distensibility Coefficient may be utilized to determine the renal artery's Incremental Modulus of Elasticity, $E_{inc}$:

$$E_{inc} = 3(1+(LCSA/IMCSA))/DC, \qquad (5)$$

where LCSA is the luminal cross-sectional area and IMCSA is the intima-media cross-sectional area:

$$LCSA = \pi D_{dia}/2)^2 \qquad (6)$$

$$IMCSA = \pi (D_{dia}/2 + IMT)^2 - LCSA \qquad (7)$$

For the renal artery, LCSA is in the range of about 7-50$mm^2$ IMCSA is in the range of about 5-80$mm^2$, and $E_{inc}$ is in the range of about 0.1-0.4 $kPa*10^3$.

For patients without significant Renal Arterial Stenosis (RAS), peak renal artery systolic blood flow velocity, $\upsilon_{max-sys}$, generally is less than about 200cm/s; while peak renal artery end-diastolic blood flow velocity, $\upsilon_{max-dia}$, generally is less than about 150cm/s, e.g., about 120cm/s.

In addition to the blood flow velocity profile of a renal artery, volumetric flow rate also is of interest. Assuming Poiseulle flow, the volumetric flow rate through a tube, $\Phi$, (often measured at the outlet of the tube) is defined as the average velocity of fluid flow through the tube, $\upsilon_{avg}$, times the cross-sectional area of the tube:

$$\Phi = \upsilon_{avg} * \pi R^2 \tag{8}$$

By integrating the velocity profile (defined in Eq. 10 above) over all r from 0 to R, it may be shown that:

$$\Phi \upsilon_{avg} * \pi R^2 = (\pi R^4 * \Delta Pr)/8\eta \Delta x \tag{9}$$

As discussed previously, for the purposes of the renal artery, $\square$ $\eta$ may be defined as $\eta_{blood}$, $\Delta x$ may be defined as $L_{RA}$, and R may be defined as $D_{RA}/2$. The change in pressure, $\Delta Pr$, across the renal artery may be measured at a common point in the cardiac cycle (e.g., via a pressure-sensing guidewire) to determine the volumetric flow rate through the renal artery at the chosen common point in the cardiac cycle (e.g. during systole and/or during enddiastole). Volumetric flow rate additionally or alternatively may be measured directly or may be determined from blood flow velocity measurements. The volumetric blood flow rate through a renal artery generally is in the range of about 500-1000 mL/min.

Thermodynamic properties of the renal artery also are of interest. Such properties include, for example, the specific heat capacity of blood and/or of the vessel wall, thermal conductivity of blood and/or of the vessel wall, thermal convectivity of blood flow past a vessel wall treatment site. Thermal radiation also may be of interest, but it is expected that the magnitude of conductive and/or convective heat transfer is significantly higher than the magnitude of radiative heat transfer.

The heat transfer coefficient may be empirically measured, or may be calculated as a function of the thermal conductivity, the vessel diameter and the Nusselt Number. The Nusselt Number is a function of the Reynolds Number and the Prandtl Number. Calculation of the Reynolds Number takes into account flow velocity and rate, as well as fluid viscosity and density, while calculation of the Prandtl Number takes into account specific heat, as well as fluid viscosity and thermal conductivity. The heat transfer coefficient of blood flowing through the renal artery is generally in the range of about 500-6000 W/m$^2$K.

An additional property of the renal artery that may be of interest is the degree of renal motion relative to the aorta, induced by respiration and/or blood flow pulsatility. A patient's kidney, located at the distal end of the renal artery, may move as much as 4 inches cranially with respiratory excursion. This may impart significant motion to the renal artery connecting the aorta and the kidney, thereby requiring from the neuromodulatory apparatus a unique balance of stiffness and flexibility to maintain contact between the thermal treatment element and the vessel wall during cycles of respiration. Furthermore, the take-off angle between the renal artery and the aorta may vary significantly between patients, and also may vary dynamically within a patient, e.g., due to kidney motion. The take-off angle generally may be in a range of about 30°-135°.

These and other properties of the renal vasculature may impose constraints upon and/or inform the design of apparatus, systems and methods for achieving renal neuromodulation via intravascular access. Specific design requirements may include accessing the renal artery, facilitating stable contact between neuromodulatory apparatus and a luminal surface or wall of the renal artery, and/or safely modulating the renal nerves with the neuromodulatory apparatus.

III. Catheter Apparatuses, Systems and Methods for Renal Neuromodulation

A. Overview

The representative embodiments provided herein include features that may be combined with one another and with the features of other disclosed embodiments. In an effort to provide a concise description of these embodiments, not all features of an actual implementation are described in the specification. It should be appreciated that in the development of any such actual implementation, as in any engineering or design project, numerous implementation-specific decisions should be made to achieve the developers' specific goals, such as compliance with system-related and business-related constraints, which may vary from one implementation to another.

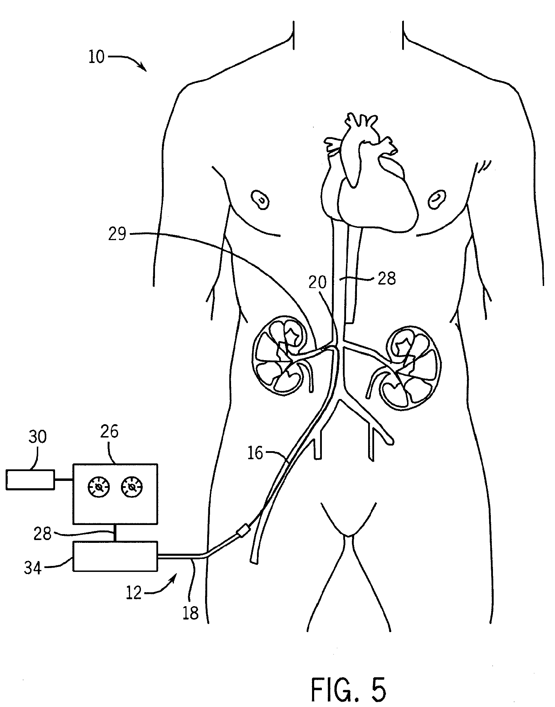

Figure 5 shows a system 10 for inducing neuromodulation of a left and/or right renal plexus (RP) through intravascular access. As just described, the left and/or right renal plexus (RP) surrounds the respective left and/or right renal artery. The renal plexus (RP) extends in intimate association with the respective renal artery into the substance of the kidney. The system induces neuromodulation of a renal plexus (RP) by intravascular access into the respective left and/or right renal artery and application of energy, such as ultrasound energy.

The system 10 includes an intravascular treatment device 12, e.g., a catheter. The treatment device 12 provides access to the renal plexus (RP) through an intravascular path that leads to a respective renal artery. The treatment device 12 includes an elongated shaft 16 having a proximal end region 18 and a distal end region 20. An ultrasound transducer 24 is disposed at or near the distal end region 20. As illustrated, the proximal end region 18 of the elongated shaft 16 is connected to a handle assembly 34. The handle assembly 34 is sized and configured to be securely or ergonomically held and manipulated by a caregiver outside an intravascular path . By manipulating the handle assembly 34 from outside the intravascular path, the caregiver may advance the elongated shaft 16 through the tortuous intravascular path, including the aorta 28 and the renal artery 29, and remotely manipulate or actuate the distal end region 20. Image guidance, e.g., CT, radiographic, IVUS, OCT or another suitable guidance modality, or combinations thereof, may be used to aid the caregiver's manipulation. The handle assembly 34 may include an actuatable element, such as a knob, pin, or lever that may control flexing of the elongated shaft 16 within the vasculature. In certain embodiments, the system 10 may also include a neutral or dispersive electrode that may be electrically connected to the generator 26 and attached to the exterior of the patient

The distal end region 20 of the elongated shaft 16 may flex in a substantial fashion to gain entrance into a respective left/right renal artery by manipulation of the elongated shaft 16. In some embodiments, the flexing may be imparted by a guide catheter, such as a renal guide catheter with a preformed or steerable bend near the distal end that directs the elongated shaft 16 along a desired path such as from an aorta to a renal artery. In other embodiments, the flexing may be imparted by a guidewire that is first delivered in to a renal artery and the elongated body 16 comprising a guidewire lumen is then passed over the guidewire in to the renal artery. Or alternatively, following insertion of a guidewire in to a renal artery a delivery sheath may be passed over a guidewire (i.e. the lumen defined by the delivery sheath slides over the guidewire) in to the renal artery. Then once the delivery sheath is placed in the renal artery the guidewire may be removed and a treatment catheter may be delivered into the renal artery. Furthermore, in particular embodiments, the flexing may be controlled via the handle assembly 34, for example by actuatable element 36 or by another control element. In particular, the flexing of the elongated shaft 16 may be accomplished as provided in U.S. Patent Application No. 12/545,648, "Apparatus, Systems, and Methods for achieving Intravascular, Thermally-Induced Renal Neuromodulation" to Wu et al.

The system 10 also includes an acoustic energy source 26 (e.g., an ultrasound energy generator). Under the control of the caregiver and/or an automated control algorithm 30, the generator 26 generates a selected form and magnitude of energy (e.g., a particular energy frequency). A cable 28 operatively attached to the handle assembly 34 electrically connects the ultrasound transducer 24 to the generator 26. At least one supply wire (not shown) passing along the elongated shaft 16 or through a lumen in the elongated shaft 16 from the handle assembly 34 to the ultrasound transducer 24 conveys the treatment energy to the ultrasound transducer 24. A control mechanism, such as a foot pedal, may be connected (e.g., pneumatically connected or electrically connected) to the generator 26 to allow the operator to initiate, terminate and, optionally, adjust various operational characteristics of the generator, including, but not limited to, power delivery.

The generator 26 may be part of a device or monitor that may include processing circuitry, such as a microprocessor, and a display. The processing circuitry may be configured to execute stored instructions relating to the control algorithm 30, The monitor may be configured to communicate with the treatment device, for example via cable 28, to control power to the ultrasound transducer 24 and/or to obtain signals from the ultrasound transducer 24 or any associated sensors. The monitor may be configured to provide vindications of power levels or sensor data, such as audio, visual or other indications, or may be configured to communicate the information to another device.

Once proximity between, alignment with, or contact between the ultrasound transducer 24 and tissue are established within the respective renal artery 29 or aorta 28, the purposeful application of energy from the generator 26 to tissue by the ultrasound transducer 24 indues one or more desired neuromodulating effects on localized regions of the renal artery and adjacent regions of the renal plexus (RP), which lay intimately within, adjacent to, or in close proximity to the adventitia of the renal artery. The purposeful application of the neuromodulating effects may achieve neuromodulation along all or a portion of the RP.

The neuromodulating effects may include application of focused ultrasound energy to achieve sustained heating, sonication, and/or cavitation. Desired thermal heating effects may include raising the temperature of target neural fibers above a desired threshold to achieve non-ablative thermal alteration, or above a higher temperature to achieve ablative thermal alteration. For example, the target temperature may be above body temperature (e.g., approximately 37°C) but less than about 45°C for non-ablative thermal alteration, or the target temperature may be about 45°C or higher for the ablative thermal alteration.

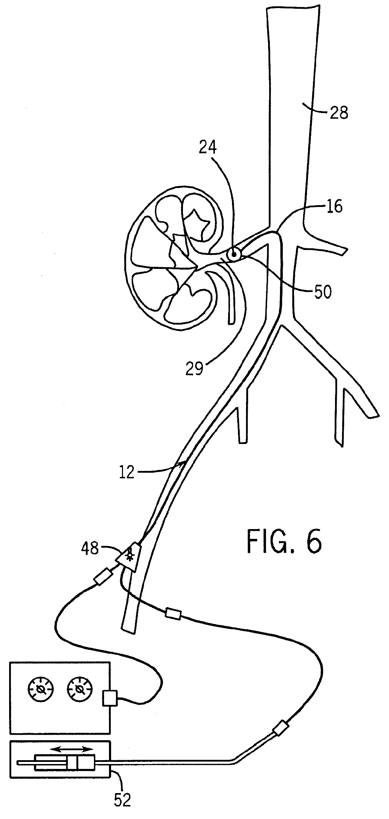

As noted, intravascular access to an interior of a renal artery may be achieved, for example, through the femoral artery, as shown in Figure 6. In particular, the elongates shaft 16 is specially sized and configured to accommodate passage through the intravascular path, which leads from a percutaneous access site in, for example, the femoral, brachial, radial, or axillary artery, to a targeted treatment site within a renal artery. In this way, the caregiver is able to orient the ultrasound transducer 24 within the aorta 28 or the renal artery 29 for its intended purpose.

The ultrasound transducer 24 may be associated with the distal region 20 of the elongated shaft 16. In particular, the distal region 20 may be steered or deflected via a steering mechanism 48 associated with the handle 34. This in turn controls the positioning of the ultrasound transducer 24 within the renal artery. As noted, because the ultrasound transducer 24 is focused at a remote point, direct contact with the arterial wall is not necessary for energy delivery. However, because energy delivery through the blood may be complex, the ultrasound transducer 24 may, in particular embodiments, be positioned against the arterial wall (i.e., in direct contact) to reduce the amount of energy that travels through the blood before reaching a desired focal point.

However, in other embodiments, the ultrasound transducer 24 may be positioned within the vasculature but not in contact with the arterial walls. In a particular embodiment, loss of acoustic energy (e.g., ultrasound energy) may be mediated by surrounding the ultrasound transducer 24 with an acoustically conductive medium, such as deaerated water. As shown, in particular embodiments, the treatment device 12 is associated with an inflatable balloon 50 that may be deployed (e.g., expanded or inflated) within the renal artery 28 so that the balloon 50 is filled with an acoustically conductive medium. The ultrasound transducer 24 is within the inflated space of the balloon 50. In the depicted embodiment, ultrasound energy travels through the conductive medium in the balloon 50, which provides a pathway for contact with the artery wall and other tissues. In addition, the balloon 50 may be oversized relative to the renal artery 28 (or, in embodiments, the aorta 29), such that the balloon 50 fills the diameter of the renal artery 28, temporarily occluding the vessel during the treatment process. In this manner, acoustic energy loss to the surroundings is minimized. The internal diameter of the renal artery is approximately 5-6 mm in an adult human. As such, a fully inflated balloon 50 may have a largest diameter of at least about 5mm, 6mm, 8mm, or 10mm. Inflation of the balloon 50 may be facilitated by inflation lumen 52, which may be associated with the catheter shaft 16. For example, the inflation lumen 52 may be formed within the shaft 16.

For practical purposes, the maximum outer dimension (e.g., diameter) of any section of the elongated shaft 16, including the ultrasound transducer/s 24 it carries and any associated structures (e.g., expandable balloon 50 or focusing structures), is dictated by the inner diameter of the guide catheter through which the elongated shaft 16 is passed. Assuming, for example, that an 8 French guide catheter (which has an inner diameter of approximately 2.312 mm (0.091 inches)) would likely be, from a clinical perspective, the largest guide catheter used to access the renal artery, and allowing for a reasonable clearance tolerance between the ultrasound transducer 24 and the guide catheter, the maximum outer dimension may be realistically expressed as being less than or equal to approximately 2.159 mm (0.085 inches). In such an embodiment, the ultrasound transducer 24 may have a contracted diameter 62 that is less than or equal to approximately 2.159 mm (0.085 inches). However, use of a smaller 5 French guide catheter may require the use of smaller outer diameters along the elongated shaft 16. For example, an ultrasound transducer 24 that is to be routed within a 5 French guide catheter would have an outer dimension of no greater than 1.347 mm (0.053 inches). In another example, an ultrasound transducer 24 to be routed within a 6 French guide catheter would have an outer dimension of no great than 1.778 mm (0.070 inches).

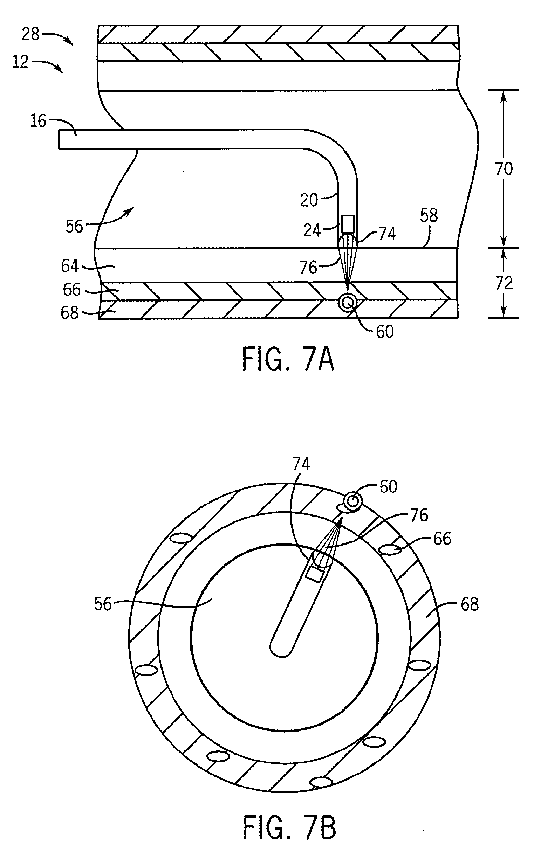

Figure 7A illustrates a catheter device 12 positioned within the interior space 56 of the renal artery 28. As noted, the ultrasound transducer 24 may be steered by a remote mechanism 48 associated with the handle 34. The ultrasound transducer 24 may be deflected within the renal artery to position the ultrasound transducer against the intima 58 according to the desired ultrasound focal point 60.

The intima 58 is the inner layer of a vessel. It consists of very thin lining of endothelial cells supported by a similarly thin layer of connective tissue. It is desirable to maintain the integrity of the intima during the treatment process, since damage may lead to stenosis. The distal region 20 may be used in conjunction with trauma reducing tip enhancements to soften the contact with the intima 58 and protect it.

In arteries, a continuous layer of elastic tissue, called the internal elastic lamina, forms the boundary between the intima 58 and the media 64. The media 64 is the middle layer of a blood vessel and in most arteries and veins it is the thickest of the three tunics. The thickness of the media 64 is generally proportional to the overall diameter of the vessel. The media consists of smooth muscle and elastic tissue in varying proportions.

The external layer 68 of the arterial wall is called adventitia. Ordinary fibrous connective tissue forms the outer layer of blood vessels. This adventitial connective tissue is usually more or less continuous with the connective tissue of the organ in which the vessel is found. That is, there is not a distinct outer boundary to the adventitia 66, and the depicted embodiment is used merely for illustrative purposes. Nevertheless, the fibers of adventitial connective tissue tend to be more concentric around the vessel and often somewhat denser than the surrounding connective tissue (fascia). The renal nerves 66 (actually multiple dispersed nerve fibers) are mostly embedded in the adventitia layer 66. Anatomic considerations for focusing the ultrasound transducer 24 onto the focal point 60 may include the diameter 70 of the renal artery and the depth of the arterial wall 72. Figure 7B shows a cross sectional view of the renal artery 28 showing the focal point 60 proximate to the renal nerves 66.

The catheter 12 at the distal region 20 is equipped with a HIFU energy transducer 24 that may be an ultrasonic crystal. The catheter 12 may include a focusing structure, such as a convex acoustic mirror in a form of a convex hemispheric cavity 74 designed to focus the sonic waves, shown as arrows 76, on the focal point 60. The geometry of the tip may be designed so that when the tip is pressed against the intima 58, the focal point 60 is in the adventitial layer 68 or even slightly beyond it (for example, in cases in which the ablation grows toward the transducer such as cases of cavitation at the focus which reflects the energy back through the near field). While the depicted treatment device 12 is configured so that the transducer 24 and the cavity 74 are coaxial 16, the focusing structure may be aligned in various configurations including an orthogonal one to the shaft to facilitate fixation of the HIFU source in the artery of the patient.

It is expected that the average ultrasound intensity for ablation of renal nerves may be in the range of 1 to 4 kW/cm$^2$ and may be delivered for a total of 10 - 60 sec to create one focal lesion. The exact best parameters for sonication may be established in a series of animal experiments for the selected design of the HIFU crystal and mirror. The selected parameters are desired to disable conduction of renal nerves for at least several months while creating minimal damage of surrounding tissue.

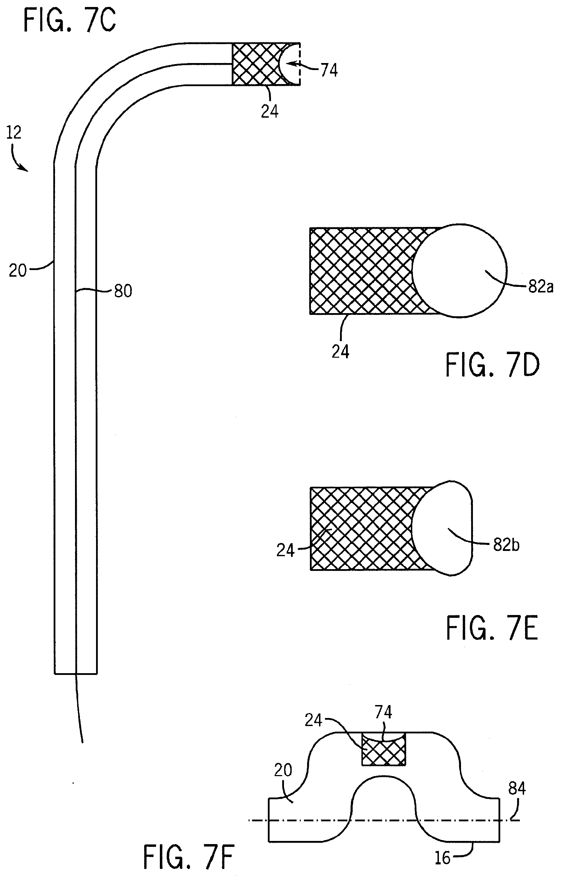

Figures 7C and 7D are alternative configurations of a distal region 20 of a treatment device 12. For example, the focusing cavity 74 may be machined or ground in a ceramic sonic crystal transducer to achieve the desired geometry to form the focal point 60. The crystal transducer 24 is mounted on the tip of the treatment device 12 and connected by electric wires 80 to the generator (e.g., generator 26, see Figure 5) that delivers electric excitation to the crystal making it vibrate with the desired frequency and intensity. To improve contact with the wall of the vessel, the focusing cavity 74 may be filled with a structure 82 formed from a material with low ultrasonic impedance, such as a thin wall water balloon or polymer. The structure may be formed in different shapes, for example as spherical shape as in Figure 7D or a semispherical shape as in Figure 7E, to improve concentration of energy on the desired area of tissue.

It is appreciated that throughout this application sonic crystals are depicted as solid cylinders but the technology is available to make them in a variety of shapes. Holes may be drilled through the crystal to allow passage of wires and fluids. Figure 7F illustrate an embodiment in which a transducer 24, e.g., a sonic mirror crystal transducer, is coupled to the treatment device so that the transducer is oriented orthogonally to the axis 84 running along the elongated shaft 16. This configuration may be advantageous for positioning the treatment device 12 correctly in the tight renal artery space. It also has potential advantage for configurations in which several transducers 24 are arranged along the length of one catheter shaft 16.

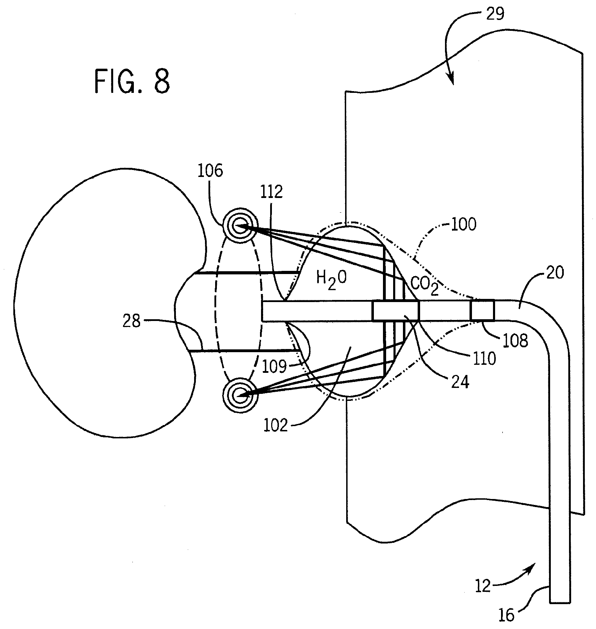

It is appreciated that several transducers 24, e.g., sonicating crystals or several cavities in one crystal, may be mounted on one treatment device to speed up energy delivery, e.g., sonication. In this case the focusing (e.g. parabolic) mirrors may be arranged in a spiral with focal axis shifted by a desired angle to create overlapping lesions. Figure 8 shows a side view of a treatment device 12 that resides mostly in the aorta 29 of the patient at the level of the branching of the renal artery 28. In the depicted embodiment, a collapsible ultrasonic reflector incorporates a gas-filled reflector balloon 100, a liquid-filled conduction balloon 102, and an ultrasonic transducer 24 disposed within the conduction balloon 102. Acoustic energy emitted by the transducer 24 is reflected by a very reflective interface between the balloons. In the renal nerve ablation procedure, the ultrasonic energy is focused into an annular focal region to ablate tissue in an annular path 106 around the ostium of the renal artery. Difference of ultrasonic impendance between the liquid and the air creates a very good sonic mirror. The balloons 100 and 102 may be made of extremely thin but strong and non stretchable polymer commonly used to make angioplasty and stent delivery balloons.

The treatment device 12 schematically depicted in Figure 8 may include, for example, a non-compliant distal balloon 102, which may be filled with a mixture of water and contrast media (e.g. in 6:1 ratio) and an integrated 1-10 MHz ultrasound crystal. A second non-compliant balloon 100, filled with carbon dioxide, forms a focusing surface (e.g. parabolic) at the base of the balloon 102. The gas-filled balloon 100 includes a proximal coupling 108 and a distal coupling 109 to the shaft 16. The liquid-filled balloon includes a proximal coupling 110 and a distal coupling 112. The distal couplings 109 and 112 may be substantially co-located on the shaft 16, while the proximal coupling 108 is more proximal that the proximal opening 110. This arrangement may create the focusing surface proximal to coupling 110. This configuration may be accomplished by a longer balloon 100 surrounding a shorter balloon 102, or, alternatively, by a single-balloon structure that includes multiple layers or compartments. Thereby, the ultrasound waves are reflected in the forward direction, focusing a ring of ultrasound energy (sonicating ring) 1-6 mm distally to the balloon surface. Treatment device 12 may be steerable through a pull wire mechanism integrated in the handle of the catheter. Several different balloon sizes may be available between 8 and 20 mm in diameter. The shaft 16 may have a central lumen used for contrast infusion into the balloon 102 and for insertion of a guide wire supporting the navigation of the treatment device 12.

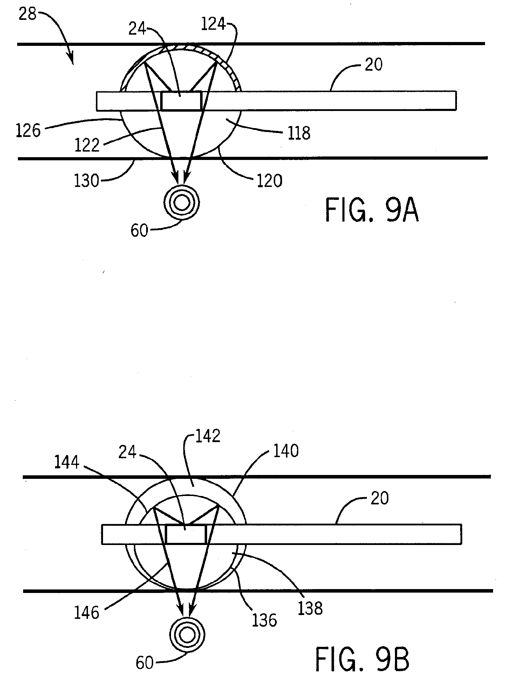

Figure 9A illustrates a treatment device 12 is equipped with a transducer 24 that emits ultrasound waves inside a balloon 120 filled with a sound-conducting medium 118 (e.g. water). Waves, depicted by arrows 122, are formed into a focal beam focusing on the focal point 60 that may be 0 to 5 mm deep in the tissue surrounding the lumen of the renal artery. As shown in Figure 9A, one hemispheric segment of the balloon incorporates material that reflects the acoustic waves 122. The material may be a coating on the surface of the balloon 120 or may be integrally formed in the material of the balloon 120. The opposing hemisphere 126 is conductive to sound and in contact with the arterial wall 130.

In Figure 9B, balloon 136, filled with conductive medium 138, is enclosed inside a balloon 140 that may be filled with a less conductive medium 142, such as gas. The reflective interface 144 between the balloons 136 and 140 creates a focusing (e.g. parabolic) mirror surface that focuses the ultrasonic waves, depicted by arrows 146. The shaft 16 may be rotated to create multiple focal points 60, e.g., overlapping regions of disabled nerves for more complete denervation. In 30-90 sec a complete nerve lesion may be achieved using this technology. It is appreciated that that periodic balloon deflations may allow blood flow to return to the renal artery. It should also be appreciated that a plurality of sonicating balloon structures (e.g., balloons 136 and 140 filled with the appropriate media and surrounding transducer 24) may be mounted on one treatment device 12 in any suitable orientation to speed up sonication. For example, the balloons may be arranged in a spiral with focal axis shifted by a desired angle to create overlapping lesions.

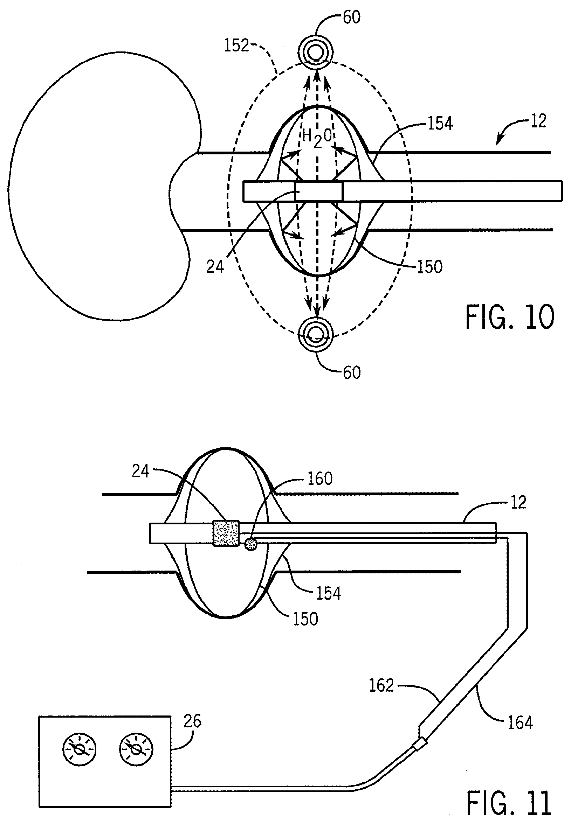

Figure 10 illustrates an embodiment in which a fluid filled balloon 150 acts as an acoustic lens and transmission media for ultrasonic energy emitted by the transducer 24. The resulting focalization forms an annular focal region 152 in the region where the conducting balloon 150 is in the contact with the wall of the renal artery. Optionally a gas filled reflecting balloon 154 may surround the conducting balloon in order to contain the energy and prevent it from escaping in the undesired directions.

Figure 11 is a system-level view of the treatment device 12 of Figure 10. The ultrasound energy may be delivered in a controlled manner to achieve desired heating of tissue in the range of 60 to 90°C. To prevent overheating and control temperature, a temperature sensor 160, such as a thermistor, is incorporated in the design of the catheter. Electric wires 162 conducting temperature signal may be incorporated into the catheter together with the excitation wires 164 that connect the ultrasonic transducer 24 to the sonic energy generator 26 that is located outside of the body. The generator 26 may be equipped with electronic circuits capable of deceiving temperature signal and controlling the energy delivered to the transducer 24. Methods well known in the control engineering may be used to maintain a user-set temperature in the balloon 150 in the desired range. It is appreciated that the temperature control feedback feature disclosed in Figure 11 may be incorporated in other designs and embodiments disclosed herein.

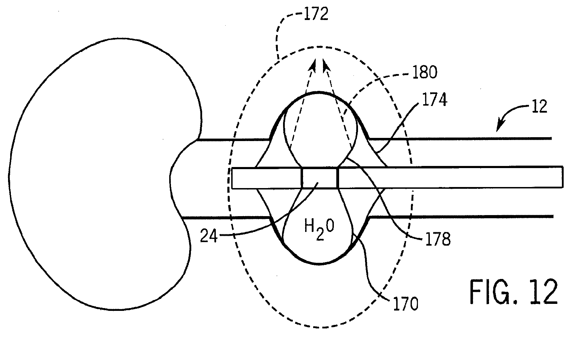

Figure 12 illustrates a fluid-filled balloon 170 that acts as an acoustic lens and transmission media for ultrasonic energy emitted by the source 24. The resulting annular focal region 172 is created where the conducting balloon 170 is in the contact with the wall of the renal artery. A gas filled reflecting balloon 174 partially surrounds the conducting balloon 170 in order to contain the energy and reduce scattering in undesired directions. The inflated conductive balloon 170 assumes an approximately toroidal shape. Since the liquid-filled torus balloon 170 is contained inside the gas filled reflecting balloon 174, the interface between the balloons creates the surface 178 that approximates the desired acoustic mirror assisting the condensing of ultrasonic energy, depicted by arrows 180, in the annular focal region 172.

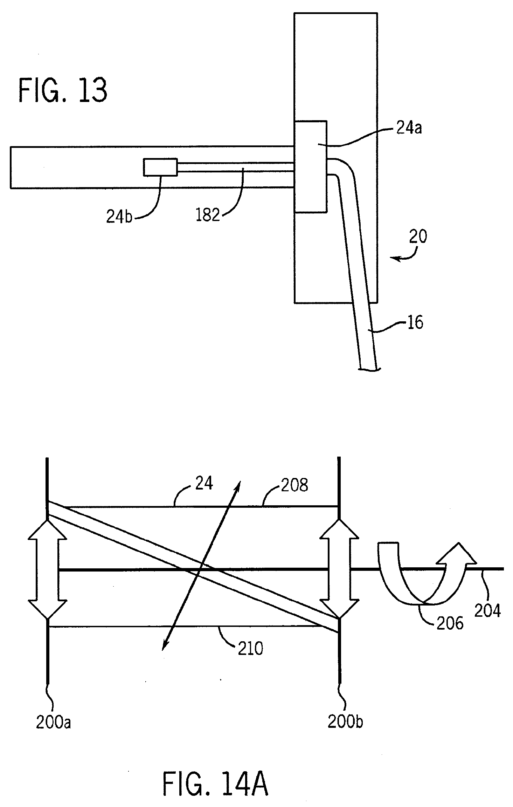

Figure 13 illustrates an embodiment in which a first transducer 24a is positioned within an aorta and a second transducer 24b is positioned inside the renal artery. The aortal transducer 24a may be positioned against the renal artery/aorta junction. One or both of the transducers 24 may be therapy transducers, imaging transducers, or a hybrid transducer, which offers both imaging and therapy.

There are many advantages to having a device with two transducers in two separate spatial locations. First, pitch-catch measurements between the transducers 24a and 24b may be used to calculate speed of sound. If the mechanical distance between 24a and 24b is know, and if a transmit event occurs on either 24a or 24b and the sound is received on the opposing transducer, then the travel time may be determined. Since the distance is known, the actual speed of sound may be determined. This information may be used to properly set delays at 24a and 24b by using time reversal processes. In this case, a small point source on either 24a or 24b is transmitted and received at the opposing transducer (e.g., transducer 24a or 24b) by a single element or multiple elements. The phase differences between the elements suggest the transmit delays required for proper focusing based on the point source location. Ideally, the point source would be positioned as close to the intended target as possible.

Since the goal is to place enough energy at the arterial wall, having a transducer near the treatment site (24b) as well as another offset transducer (24a), allows for measurement of the power near the treatment site. This calibration measurement may be used to adjust the treatment power to achieve the required therapeutic effect. It may also be used to determine the therapy beam geometry. In addition, the arrangement of the transducers 24a and 24b may be selected to properly position each transducer 24 within the appropriate vascular region. For example, relative to an aortic transducer 24a configured to be positioned at a renal artery/aorta junction, the renal artery transducer 24b may be spaced at least 5mm distally along the elongated shaft to allow the transducer 24b to fully enter the renal artery. The distance between the transducers 24a and 24b may be selected with patient anatomy in mind. It may be advantageous to position a renal artery transducer at particular locations along the renal artery (e.g., at around a mid-point of the renal artery) to achieve maximum therapeutic benefit.

If 24a and 24b are used to generate image data, it is possible to compound images of the potential treatment site, which leads to superior image contrast. Different types of imaging may be used to help locate the treatment site. Possible imaging modes between the two transducers include: Compound B-mode, C-mode with both magnitude and direction information, C-mode from acoustic streaming, Compound Power Doppler, elasticity imaging between two transducers.

In addition to the pretreatment advantages of the two transducer design, it also offers advantages during treatment. For example, if one transducer is used for therapy, then the other transducer may be used for imaging. Synchronization between the two systems allows the imaging system to produce images when therapy is off as well as potentially image the therapy application when therapy is on. This allows changes in tissue characteristics during treatment to be visualized through regular B-mode imaging, elasticity imaging, shear wave imaging or temperature estimates. Again if one transducer is used as the imaging transducer, then tissue movement may be tracked to give feedback to the therapy transducer so the beam stays within the treatment zone.

Although it is possible to therapeutically treat the renal nerve with either 24a or 24b, it is also advantageous to possibly combine the power from the transducers to increase the localization of the lesion. Typically, focused transducers produce elongated (e.g., cigar-like) lesions. It may be beneficial given the treatment zone size to produce lesions that are spherical. This may be achieved by combining therapy beams from multiple transducers. For example, 24a and 24b could simultaneously deliver energy to the arterial wall.

The transducer (24a or 24b) may by a single element or multi-element transducer that is side looking or forward looking. In addition to these designs, 24b may also image and deliver therapy. As shown in Figure 14A, the transducer 24, which may be any suitable shape, such as cylindrical, rectangular, or elliptical, may have at least some degree of freedom along axis 200 to allow for vertical deflection within the renal artery (e.g., deflection along a diameter of the renal artery relative to the elongated shaft 16). In addition, the transducer 24 may have rotational freedom about an axis 204 of the elongated shaft. That is, the transducer 24 may rotate as illustrated by arrow 206. The tilt or rotation may be controlled by a steering mechanism (e.g., mechanism 48 associated with the handle assembly 34, see Figure 6). The transducer tilt increases the spread of the lesion, shown by arrows 209, so that manual movement is not required. To facilitate such tilting, a portion 182 of the distal region 20 of the elongated shaft 16 between the transducers 24a and 24b may be more flexible than other regions of the elongated shaft 16. In addition, greater flexibility of the portion 182 may allow the renal artery transducer to move along with the natural movement of the renal artery. In other embodiments, the portion 182 may be generally as flexible as the distal region 20 of the elongated shaft 16.

In particular embodiments, energy emanates from the top surface 208 and the bottom surface 210 of the transducer 24. This increases thermal deposition rate so the lesion is completed sooner. In particular embodiments, an aortic transducer 24a may be sized to accommodate the relatively larger aorta while the renal artery transducer 24b may be relatively smaller to fit within the renal artery. In addition, the aortic transducer 24a may be sized to fully or partially occlude the renal artery/aorta junction. As such, at least one dimension of the transducer 24a may be larger than a renal artery diameter (e.g., larger than about 5mm-6mm).

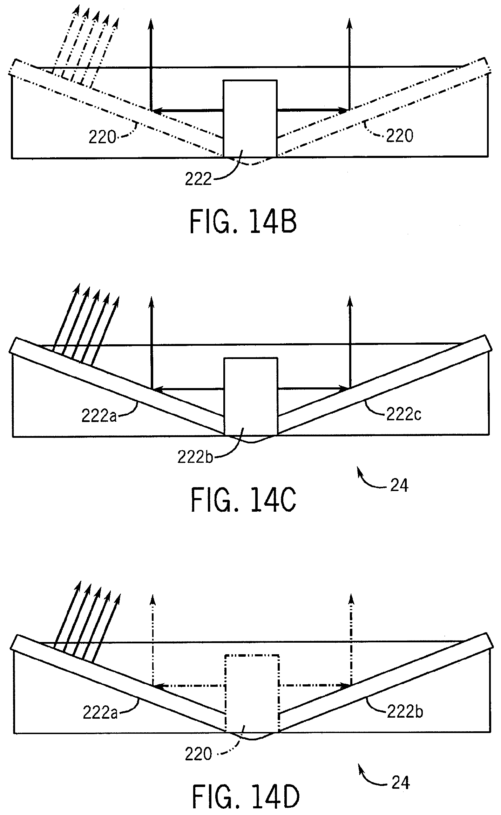

Figure 14B illustrates an embodiment in which a transducer 24 is capable of imaging as well as delivering therapy. The imaging portion 220 of the transducer 24 may be a single element or multi-element transducer, as shown. The imaging transducer may be mechanically focused in the piezoelectric material or through a lens. The imaging transducer may designed in such a way that it is highly reflective to the therapy frequency yet transparent to the imaging frequency. The shape of the imaging transducer may be used to focus the reflected therapy energy. This may be achieved by proper choice of acoustic materials, impendance and thickness, as well as the design of the electrical circuit connected to the imaging transducer. The therapy portion 222 of the transducer 24 may be a single element or multi-element transducer that is a partial cylinder or full cylinder with a mechanical focus in the height and/or circumferential direction.

Figure 14C is an alternative embodiment in the imaging portion 220 of the transducer 24 is replaced by additional therapy transducer 222. This design increases the available transducer active area, which is directly correlated to focal gain and ability to thermally heat tissue. The portions 222a and 222c may be used to refocus the energy from portion 222b as well as focus its energy at the arterial wall. In both cases, the therapy transducer portions 222 may be single element or multiple element transducers,

Figure 14D shows yet another version where the portion 220, disposed between portions 222a and 222b, is an imaging transducer. If the portion 220 is configured to move relative to portions 222a and 222b, then a multi-dimensional image may be generated. In this case, the therapy transducer portion 220 is designed to be highly reflective to the imaging frequency.

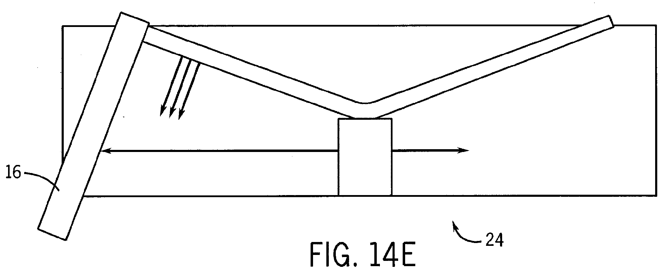

It is also possible to change the slant of the transducer from concave to convex and still achieve similar results, as shown in Figure 14E. In particular, the depicted embodiment may be tilted or slanted relative to the elongated shaft 16, depending on the desired focal point. Further, in other embodiments, individual portions of a transducer 24, e.g., 222a, 222b, and 222c, may all be configured to be articulated and to have at least one degree of freedom relative to one another. The transducer may include a mirror or other focusing structure 223 to direct the ultrasound energy, shown by arrows 225 and 227

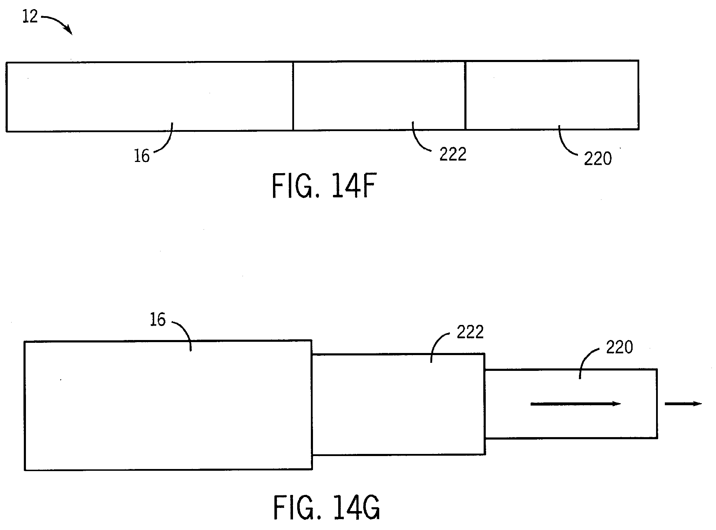

Figure 14F illustrates an embodiment in which both the therapy portion 222 and imaging portion 220 are adjacent to each other along the elongated shaft. In a specific embodiment, shown in FIG. 14G, the imaging transducer portion 220 may be capable of sliding past the therapy transducer portion 222 after placement in the vasculature. In such embodiments, the imaging portion 220 and the therapy portion 222 may be coaxially aligned to facilitate the movement.

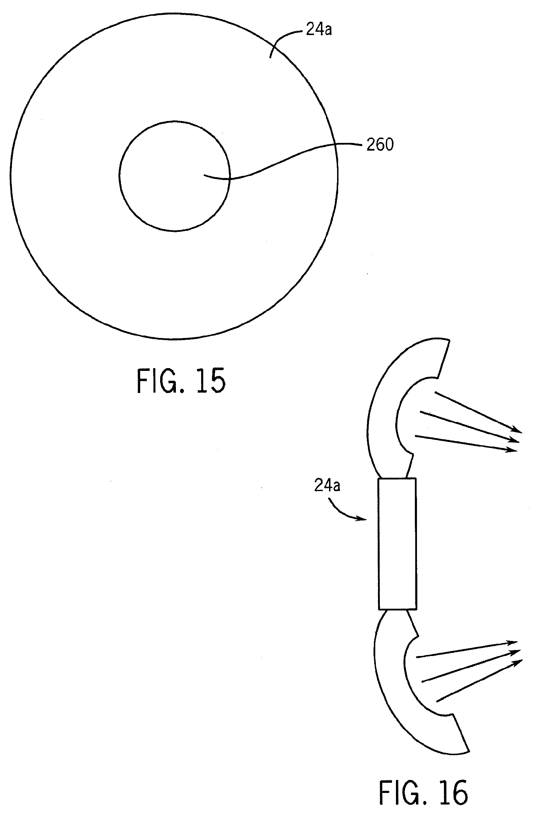

As noted, it is contemplated that positioning an ultrasound transducer 24 within the aorta may provide certain benefits. The aortic transducer (e.g., 24a) could be a focused piston, a 1D or multiD linear array (one sided or two sided around the aorta/renal artery junction), or a ring transducer. The aorta transducer 24a may consist of an imaging transducer or a therapy transducer with a single element or multi-elements. Figure 15 shows a cross-sectional view of a transducer 24 that is generally piston-like. A passageway 260 through the transducer 24a accommodates the distal region 20 of the elongated shaft and associated transducer 24b. In a specific embodiment, the distance between transducers 24a and 24b (not shown) may be adjusted by sliding a portion of the elongated shaft through the passageway 260, to either increase or decrease the distance between the two transducers 24a and 24b. The distance may be adjusted depending on a particular patient's anatomy or to change the location of a focal point 60.

In addition, if the transducer 24a is a focused piston, the transducer 24a can be centered on the renal artery transducer 24b by using pitch-catch techniques. For example, splitting the transducer 24a into four quadrants would allow acoustic timing differences to determine the distance to the transducer 24b. Once the transducer 24a is centered on the transducer 24b, which means it is centered on the artery, therapy may be applied in such a way to just heat the outer part of the artery. This could be accomplished through a combination heating approach with the transducer 24b or by cooling the interior location of the renal artery while heating the outside with the acoustic beam. Since the transducer 24a is a circular transducer, the lesion would be circularly symmetric and possibly reduce the overall treatment time by treating the entire perimeter simultaneously. Instead of a single focus, the transducer 24a may also have a focus that produces a ring. The transducer 24a may be tilted with a degree of mechanical curvature in the radial direction as shown in Figure 16.

The transducer 24a may also include imaging or targeting modalities. This may be accomplished by using a fully synthetic aperture (transmit and receive). In this case, the ring transducer may generate volume images of the renal artery to assist with proper placement of the therapy transducer (the transducer 24b).

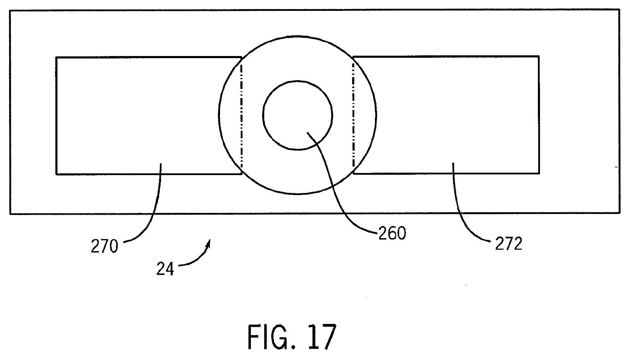

Figure 17 illustrates an embodiment in which a transducer 24 is made up of two separate transducers, 270 and 272. These two transducers could be an imaging/targeting transducer or a therapy transducer. If both are imaging transducers, then compound images of the renal artery may be acquired. If both are imagine transducers, then the therapy beams may be overlapped to improve the containment of the lesion in the adventitia of the renal artery.

B. Size and Configuration of the HIFU Focal Zones for Achieving Neuromodulation in a Renal Artery

It should be understood that the embodiments provided herein may be used in conjunction with one or more ultrasound transducers 24. In some patients, it may be desirable to use the ultrasound transducer(s) 24 to create a single lesion or multiple focal lesions that are circumferentially spaced along the longitudinal axis of the renal artery. A single focal lesion with desired longitudinal and/or circumferential dimensions, one or more full-circle lesions, multiple circumferentially spaced focal lesions at a common longitudinal position, and/or multiple longitudinally spaced focal lesions at a common circumferential position alternatively or additionally may be created.

Depending on the size, shape, and number of the ultrasound transducers 24, the lesions may be circumferentially spaced along the longitudinal axis of the renal artery. In particular embodiments, it is desirable for each lesion to cover at least 10% of the vessel circumference to increase the probability of affecting the renal plexus. It is also desirable that each lesion be positioned into and beyond the adventitia to thereby affect the renal plexus. However, lesions that are too deep (e.g., >5mm) run the risk of interfering with non-target tissue and tissue structures (e.g., renal vein) so a controlled depth of energy treatment is also desirable.

In certain embodiments, a plurality of focal zones of the ultrasound transducer 24 may be used during treatment. Refocusing the ultrasound transducer 24 in both the longitudinal and angular dimensions provides a second treatment site for treating the renal plexus. Energy then may be delivered via the ultrasound transducer to form a second focal lesion at this second treatment site, thereby creating a second treatment zone. For embodiments in which multiple ultrasound transducers 24 are associated with the catheter 16, the initial treatment may result in two or more lesions, and refocusing may allow additional lesions to be created.

In certain embodiments, the lesions created via refocusing of the ultrasound transducer 24 are angularly and longitudinally offset from the initial lesion(s) about the angular and lengthwise dimensions of the renal artery, respectively. Superimposing the lesions created by initial application and repositioning, may result in a discontinuous (i.e., the lesion is formed from multiple, longitudinally and angularly spaced treatment zones) lesion. One or more additional focal lesions optionally may be formed via additional refocusing of the ultrasound transducer 24. In one representative embodiment, superimposition of all or a portion of the lesions provides a composite treatment zone that is non-continuous (i.e., that is broken up along the lengthwise dimension or longitudinal axis of the renal artery), yet that is substantially circumferential (i.e., that substantially extends all the way around the circumference of the renal artery over a lengthwise segment of the artery).

C. Applying Energy to Tissue Via the Ultrasound Transducer

Referring back to Figure 5, in the illustrated embodiment, the generator 26 may supply energy to the ultrasound transducer 24 to generate acoustic waves. Energy delivery may be monitored and controlled, for example, via data collected with one or more sensors, such as temperature sensors (e.g., thermocouples, thermistors, etc.), impedance sensors, pressure sensors, optical sensors, flow sensors, chemical sensors, etc., which may be incorporated into or on the ultrasound transducer 24 and/or in/on adjacent areas on the distal end region 20. A sensor may be incorporated into the ultrasound transducer 24 in a manner that specifies whether the sensor(s) are in contact with tissue at the treatment site and/or are facing blood flow. The ability to specify sensor placement relative to tissue and blood flow is highly significant, since a temperature gradient across the electrode from the side facing blood flow to the side in contact with the vessel wall may be up to about 15°C. Significant gradients across the electrode in other sensed data (e.g., flow, pressure, impedance, etc.) also are expected.

The sensor(s) may, for example, be incorporated on the side of the ultrasound transducer 24 that contacts the vessel wall at the treatment site during power and energy delivery or may be incorporated on the opposing side of the ultrasound transducer 24 that faces blood flow during energy delivery, and/or may be incorporated within certain regions of the ultrasound transducer 24 (e.g., distal, proximal, quandrants, etc.). In some embodiments, multiple sensors may be provided at multiple positions along the ultrasound transducer 24 or elongated shaft 16 and/or relative to blood flow. For example, a plurality of circumferentially and/or longitudinally spaced sensors may be provided. In one embodiment, a first sensor may contact the vessel wall during treatment, and a second sensor may face blood flow.

Additionally or alternatively, various microsensors may be used to acquire data corresponding to the ultrasound transducer, the vessel wall and/or the blood flowing across the ultrasound transducer. For example, arrays of micro thermocouples and/or impedance sensors may be implemented to acquire data along the ultrasound transducer or other parts of the treatment device. Sensor data may be acquired or monitored prior to, simultaneous with, or after the delivery of energy or in between pulses of energy, when applicable. The monitored data may be used in a feedback loop to better control therapy, e.g., to determine whether to continue or stop treatment, and it may facilitate controlled delivery of an increased or reduced power or a longer or shorter duration therapy.

### D. Cooling the Ultrasound Transducer

Non-target tissue may be protected by blood flow (F) within the respective renal artery that serves as a conductive and/or convective heat sink that carries away excess thermal energy. In particular embodiments, since blood flow (F) is not blocked by the elongated shaft 16 and the ultrasound transducer 24, the native circulation of blood in the respective renal artery serves to remove excess thermal energy from the non-target tissue and the ultrasound transducer. The removal of excess thermal energy by blood flow also allows for treatments of higher power, where more power may be delivered to the target tissue as heat is carried away from the application site and non-target tissue. In this way, intravascularly-delivered ultrasound energy heats target neutral fibers located proximate to the vessel wall to modulate the target neutral fibers, while blood flow (F) within the respective renal artery protects non-target tissue of the vessel wall from excessive or undesirable thermal injury. In particular, because HIFU may employ remote focal points, the highest temperature treatment regions may be located outside of or on an exterior surface of a renal artery.

It may also be desirable to provide enhanced cooling by inducting additional native blood flow across the ultrasound transducer 24. For example, techniques and/or technologies may be implemented by the caregiver to increase perfusion through the renal artery or to the ultrasound transducer itself. These techniques include positioning partial occlusion elements (e.g., balloons) within upstream vascular bodies such as the aorta, or within a portion of the renal artery to improve flow across the ultrasound transducer.

In addition, or as an alternative, to passively utilising blood flow (F) as a heat slink, active cooling may be provided to remove excess thermal energy and protect non-target tissues. For example, a thermal fluid infusate may be injected, infused, or otherwise delivered into the vessel in an open circuit system. Thermal fluid infusates used for active cooling may, for example, include (room temperature or chilled) saline or some other biocompatible fluid. The thermal fluid infusate(s) may, for example, be introduces through the treatment device 12 via one or more infusion lumens and/or ports. When introduced into the bloodstream, the thermal fluid infusate(s) may, for example, be introduced through a guide catheter at a location upstream from the ultrasound transducer 24 or at other locations relative to the tissue for which protection is sought. In a particular embodiment fluid infusate is injected through a lumen associated with the elongated shaft 16 so as to flow around ultrasound transducer 24. The delivery of a thermal fluid infusate in the vicinity of the treatment site (via an open circuit system and/or via a closed circuit system) may, for example, allow for the application of increased/higher power, may allow for the maintenance of lower temperature at the vessel wall during energy delivery, may facilitate the creation of deeper or larger lesions, may facilitate a reduction in treatment time, may allow for the use of a smaller transducer size, or a combination thereof.

Accordingly, the treatment device 12 may include features for an open circuit cooling system, such as a lumen in fluid communication with a source of infusate and a pumping mechanism (e.g., manual injection or a motorized pump) for injection or infusion of saline or some other biocompatible thermal fluid infusate from outside the patient, through elongated shaft 16 and towards the ultrasound transducer 24 into the patient's bloodstream during energy delivery. In addition, the distal end region 20 of the elongated shaft 16 may include one or more ports for injection or infusion of saline directly at the treatment site. Further, such a system may also be used in conjunction with an ultrasound transducer 24 that is positioned inside one or more inflatable balloons.

IV. Use of the System

A. Intravascular Delivery, Deflection and Placement of the Treatment Device

any one of the embodiments of the treatment devices 12 described herein may be delivered over a guide wire using conventional over-the-wire techniques. When delivered in this manner, the elongated shaft 16 includes a passage or lumen accommodating passage of a guide wire.

Alternatively, any one of the treatment devices 12 described herein may be deployed using a conventional guide catheter or pre-curved renal guide catheter (e.g., as shown in Figure 12). When using a guide catheter, the femoral artery is exposed and cannulated at the base of the femoral triangle, using conventional techniques. In one exemplary approach, a guide wire is inserted through the access site and passed using image guidance through the femoral artery, into the iliac artery and aorta, and into either the left or right renal artery. A guide catheter may be passed over the guide wire into the accessed renal artery. The guide wire is then removed. Alternatively, a renal guide catheter, which is specifically shaped and configured to access a renal artery, may be used to avoid using a guide wire. Still alternatively, the treatment device may be routed from the femoral artery to the renal artery using angiographic guidance and without the need of a guide catheter.

When a guide catheter is used, at least three delivery approaches may be implemented. In one exemplary approach, one or more of the aforementioned delivery techniques may be used to position a guide catheter within the renal artery just distal to the entrance of the renal artery. The treatment device is then routed via the guide catheter into the renal artery. Once the treatment device is properly positioned within the renal artery, the guide catheter is retracted from the renal artery into the abdominal aorta. In this approach, the guide catheter should be sized and configured to accommodate passage of the treatment device. For example, a 6 French guide catheter may be used.

In a second exemplary approach, a first guide catheter is placed at the entrance of the renal artery (with or without a guide wire). A second guide catheter (also called a delivery sheath) is passed via the first guide catheter (with or without the assistance of a guide wire) into the renal artery. The treatment device is then routed via the second guide catheter into the renal artery. Once the treatment device is properly positioned within the renal artery the second guide catheter is retracted, leaving the first guide catheter at the entrance to the renal artery. In this approach the first and second guide catheters should be sized and configured to accommodate passage of the second guide catheter within the first guide catheter (i.e., the inner diameter of the first guide catheter should be greater than the outer diameter of the second guide catheter). For example, the first guide catheter could be 8 French in size and the second guide catheter could be 5 French in size.

In a third exemplary approach, a renal guide catheter is positioned within the abdominal aorta, just proximal to the entrance of the renal artery. The treatment device 12 as described herein is passed through the guide catheter and into the accessed renal artery. The elongated shaft makes atraumatic passage through the guide catheter, in response to forces applied to the elongated shaft 16 through the handle assembly 34.

B. Control of Applied Energy

With the treatments disclosed herein for delivering therapy to target tissue, it may be beneficial for energy to be delivered to the target neural structures in a controlled manner. The controlled delivery of energy will allow the zone of thermal treatment to extend into the renal fascia while reducing undesirable energy delivery or thermal effects to the vessel wall. A controlled delivery of energy may also result in a more consistent, predictable and efficient overall treatment. Accordingly, the generator 26 desirably includes a processor-based control including a memory with instructions for executing an algorithm 30 (see Figure 5) for controlling the delivery of power and energy to the energy delivery device. The algorithm 30, a representative embodiment of which is shown in Figure 43, may be implemented as a conventional computer program for execution by a processor coupled to the generator 26. A caregiver using step-by-step instructions may also implement the algorithm 30 manually.

The operating parameters monitored in accordance with the algorithm may include, for example, temperature, time, impedance, power, flow velocity, volumetric flow rate, blood pressure, heart rate, etc. Discrete values in temperature may be used to trigger changes in power or energy delivery. For example, high values in temperature (e.g. 85°C) could indicate tissue desiccation in which case the algorithm may decrease or stop the power and energy delivery to prevent undesirable thermal effects to target or non-target tissue. Time additionally or alternatively may be used to prevent undesirable thermal alteration to non-target tissue. For each treatment, a set time (e.g., 2 minutes) is checked to prevent indefinite delivery of power.

Impedance may be used to measure tissue changes. In particular embodiments, when ultrasound energy is applied to the treatment site, the impedance will decrease as the tissue cells become less resistive to current flow. If too much energy is applied, tissue desiccation or coagulation may occur near the electrode, which would increase the impedance as the cells lose water retention and/or the electrode surface area decreases (e.g., via the accumulation of coagulum). Thus, an increase in tissue impedance may be indicative or predictive of undesirable thermal alteration to target or non-target tissue. In other embodiments, the impedance value may be used to assess contact of the ultrasound transducer 24 with the tissue. For a dual electrode configuration (e.g., when the ultrasound transducer(s) 24 includes two or more electrodes,) a relatively small and stable impedance value may be indicative of good contact with the tissue. For a single electrode configuration, a stable value may be indicative of good contact. Accordingly, impedance information may be provided to a downstream monitor, which in turn may provide an indication to a caregiver related to the quality of the ultrasound transducer 24 contact with the tissue.

Additionally or alternatively, power is an effective parameter to monitor in controlling the delivery of therapy. Power is a function of voltage and current. The algorithm may tailor the voltage and/or current to achieve a desired ultrasound profile.

Derivatives of the aforementioned parameters (e.g., rates of change) also may be used to trigger changes in power or energy delivery. For example, the rate of charge in temperature could be monitored such that power output is reduced in the event that a sudden rise in temperature is detected. Likewise, the rate of change of impedance could be monitored such that power output is reduced in the event that a sudden rise in impedance is detected.

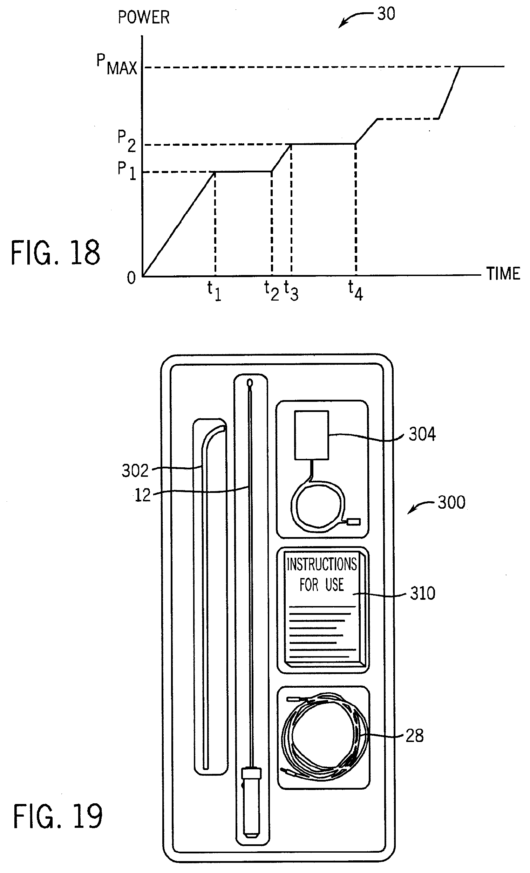

As seen in Figure 18, when a caregiver initiates treatment (e.g., via the foot pedal), the control algorithm 30 includes instructions to the generator 26 to gradually adjust its power output to a first power level $P_1$ over a first time period $t_1$ (e.g., 15 seconds). The power increase during the first time period is generally linear. As a result, the generator 26 increases its power output at a generally constant rate of $P_1/t_1$. Alternatively, the power increase may be non-linear (e.g., exponential or parabolic) with a variable rate of increase. Once $P_1$ and $t_1$ are achieved, the algorithm may hold at $P_1$ until a new time $t_2$ for a predetermined period of time $t_2 - t_1$ (e.g., 3 seconds). At $t_2$ power is increased by a predetermined increment (e.g., 1 watt) to $P_2$ over a predetermined period of time, $t_3 - t_2$ (e.g., 1 second). This power ramp in predetermined increments of about 1 watt over predetermined periods of time may continue until a maximum power $P_{MAX}$ is achieved or some other condition is satisfied. Optionally, the power may be maintained at the maximum power $P_{MAX}$ for a desired period of time or up to the desired total treatment time (e.g., up to about 120 seconds).

In Figure 18, algorithm 30 illustratively includes a power-control algorithm. However, it should be understood that algorithm 30 alternatively may include a temperature-control algorithm. For example, power may be gradually increased until a desired temperature (or temperatures) is obtained for a desired duration (durations). In another embodiment, a combination power-control and temperature-control algorithm may be provided.

As discussed, the algorithm 30 includes monitoring certain operating parameters (e.g., temperature, time, impendance, power, flow velocity, volumetric flow rate, blood pressure, heart rate, etc.). The operating parameters may be monitored continuously or periodically. The algorithm 30 checks the monitored parameters against predetermined parameter profiles to determine whether the parameters individually or in combination fall within the ranges set by the predetermined parameter profiles. If the monitored parameters fall within the ranges set by the predetermined parameter profiles, then treatment may continue at the commended power output. If monitored parameters fall outside the ranges set by the predetermined parameter profiles, the algorithm 30 adjusts the commanded power output accordingly. For example, if a target temperature (e.g., 65°C) is achieved, then power delivery is kept constant until the total treatment time (e.g., 120 seconds) has expired. If a first temperature threshold (e.g., 70°C) is achieved or exceeded, then power is reduced in predetermined increments (e.g., 0.5 watts, 1.0 watts, etc.) until a target temperature is achieved. If a second power threshold (e.g., 85°C) is achieved or exceeded, thereby indicating an undesirable condition, then power delivery may be terminated. The system may be equipped with various audible and visual alarms to alert the operator of certain conditions.

The following is a non-exhaustive list of events under which algorithm 30 may adjust and/or terminate/discontinue the commanded power output:

(1) The measured temperature exceeds a maximum temperature threshold (e.g., about 70 to about 85°C.).

(2) The average temperature derived from the measured temperature exceeds an average temperature threshold (e.g., about 65°C.).

(3) The rate of change of the measured temperature exceeds a rate of change threshold.

(4) The temperature rise over a period of time is below a minimum temperature change threshold while the generator 26 has non-zero output. Poor contact between the ultrasound transducer 24 and the arterial wall may cause such a condition.

(5) A measured impedance exceeds an impedance threshold (e.g., <20 Ohms, or >500 Ohms).

(6) A measured impedance exceeds a relative threshold (e.g., impedance decreases from a starting or baseline value and then rises above this baseline value)

(7) A measured power exceeds a power threshold (e.g., >8 Watts or >10 Watts).

(8) A measured duration of power delivery exceeds a time threshold (e.g., >120 seconds).

Advantageously, the magnitude of maximum power delivered during renal neuromodulation treatment in accordance with the present disclosure may be relatively low as compared, for example, to the power levels utilized in electrophysiology treatments to achieve cardiac tissue transmural lesions. Since relatively low power levels may be utilized to achieve such renal neuromodulation, the flow rate and/or total volume of intravascular infusate injection needed to maintain the ultrasound transducer and/or non-target tissue at or below a desired temperature during power delivery (e.g., at or below about 50°C, for example, at or below about 45°C) also may be relatively lower than would be required at the higher power levels used, for example, in electrophysiology HIFU treatments. In embodiments in which active cooling is used, the relative reduction in flow rate and/or total volume of intravascular infusate infusion advantageously may facilitate the use of intravascular infusate in higher risk patient groups that would be contraindicated were higher power levels and, thus, correspondingly higher infusate rates/volumes utilized (e.g., patients with heart disease, heart failure, renal insufficiency and/or diabetes mellitus).

V. <u>Prepackaged Kit for Distribution, Transport and Sale of the Disclosed Apparatuses and Systems</u>

As shown in Figure 19, one or more components of the system 10 shown in Figure 5 may be packaged together in a kit 300 for convenient delivery to and use by the customer/clinical operator. Components suitable for packaging include the treatment device 12, the cable 28 for connecting the treatment device 12 to the generator 26, and one or more guide catheters 302 (e.g., a renal guide catheter), and a neutral or dispersive electrode 304. Cable 28 may also be integrated into the treatment device 12 such that both components are packaged together. Each component may have its own sterile packaging (for components requiring sterilization) or the components may have dedicated sterilized compartments within the kit packaging. This kit may also include step-by-step instructions 310 for use that provide the operator with technical product features and operating instructions for using the system 10 and treatment device 12, including all methods of insertion, delivery, placement, and use of the treatment device 12 disclosed herein.

VI. <u>Additional Clinical Uses of the Disclosed Apparatuses, Methods and Systems</u>

Although certain embodiments of the present techniques relate to at least partially denervating a kidney of a patient to block afferent and/or efferent neural communication from within a renal blood vessel (e.g., renal artery), the apparatuses, methods and systems described herein may also be used for other intravascular treatments. For example, the aforementioned catheter system, or select aspects of such system, may be placed in other peripheral blood vessels to deliver energy and/or electric fields to achieve a neuromodulatory affect by altering nerves proximate to these other peripheral blood vessels. There are a number of arterial vessels arising from the aorta which travel alongside a rich collection of nerves to target organs. Utilizing the arteries to access and modulate these nerves may have clear therapeutic potential in a number of disease states. Some examples include the nerves encircling the celiac trunk, superior mesenteric artery, and inferior mesenteric artery.

Sympathetic nerves proximate to or encircling the arterial blood vessel known as the celiac trunk may pass through the celiac ganglion and follow branches of the celiac trunk to innervate the stomach, small intestine, abdominal blood vessels, liver, bile ducts, gallbladder, pancreas, adrenal grands, and kidneys. Modulating these nerves either in whole (or in part via selective modulation) may enable treatment of conditions including (but not limited to) diabetes, pancreatitis, obesity, hypertension, obesity related hypertension, hepatitis, hepatorenal syndrome, gastric ulcers, gastric motility disorders, irritable bowel syndrome, and autoimmune disorders such as Chron's disease.

Sympathetic nerves proximate to or encircling the arterial blood vessel known as the inferior mesenteric artery may pass through the inferior mesenteric ganglion and follow branches of the inferior mesenteric artery to innervate the colon, rectum, bladder, sex organs, and external genitalia. Modulating these nerves either in whole (or in part via selective modulation) may enable treatment of conditions including (but not limited to) GI motility disorders, colitis, urinary retention, hyperactive bladder, incontinence, infertility, polycystic ovarian syndrome, premature ejaculation, erectile dysfunction, dyspareunia, and vaginismus.

While arterial access and treatments have received been provided herein, the disclosed apparatuses, methods and systems may also be used to deliver treatment from within a peripheral vein or lymphatic vessel.

VII. Conclusion

The above detailed descriptions of embodiments of the disclosure are not intended to be exhaustive or to limit the disclosure to the precise form disclosed above. Although specific embodiments of, and examples for, the disclosure are described above for illustrative purposes, various modifications are possible within the scope of the disclosure, as those skilled in the relevant art will recognize. For example, while steps are presented in a given order, alternative embodiments may perform steps in a different order. The various embodiments described herein may also be combined to provide further embodiments.

From the foregoing, it will be appreciated that specific embodiments of the disclosure have been described herein for purposes of illustration, but well-know structures and functions have not been shown or described in detail to avoid unnecessarily obscuring the description of the embodiments of the disclosure. Where the context permits, singular or plural terms may also include the plural or singular term, respectively. For example, much of the disclosure herein describes an ultrasound transducer 24 (e.g., an electrode) in the singular. It should be understood that this application does not exclude two or more ultrasound transducers or electrodes

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. From the foregoing, it will also be appreciated that specific embodiments have been described herein for purposes of illustration, but that various modifications may be made without deviating from the disclosure. Accordingly, the disclosure is not limited except as by the appended claims.

**Claims**

1. A catheter apparatus for intravascular modulation of renal nerves, the catheter apparatus comprising:

    an elongated shaft (16) having a proximal portion (18) and a distal portion (20), the distal portion (20) of the shaft (16) configured for intravascular delivery to a renal artery of a patient;
    a first ultrasound transducer (24a) associated with the distal portion (20) of the shaft (16) and configured to be positioned within an abdominal aorta; and
    a second ultrasound transducer (24b) associated with the distal portion (20) of the shaft (16) such that the second ultrasound transducer (24b) is spaced apart about 5mm to about 10cm from the first transducer (24a) in a distal direction, and wherein the second ultrasound transducer (24b) is configured to be positioned within the renal artery while the first ultrasound transducer (24a) is positioned in the abdominal aorta,
    wherein the first ultrasound transducer is configured to be positioned within an abdominal aorta when energy is delivered to the arterial wall by either the first ultrasound transducer (24a) or the second ultrasound transducer (24b) or simultaneously by the first ultrasound transducer (24a) and the second ultrasound transducer (24b).

2. The catheter apparatus of claim 1 wherein the elongated shaft (16) comprises a lumen configured to receive a guide wire.

3. The catheter apparatus of claim 1 wherein the elongated shaft (16), the first ultrasound transducer (24a), and the second ultrasound transducer (24b) are sized and configured for intravascular delivery via a 6 French (2 mm) or smaller guide catheter.

4. The catheter apparatus of claim 1 wherein at least one of the first ultrasound transducer (24a) or the second ultrasound transducer (24b) comprises an imaging transducer.

5. The catheter apparatus of claim 1 wherein the first ultrasound transducer (24a) and the second ultrasound transducer (24b) comprise therapy transducers.

6. The catheter apparatus of claim 1, further comprising a source of cooling infusate coupled to a delivery lumen in the elongated shaft, wherein the delivery lumen comprises an opening located in the distal portion (20) of the elongated shaft (16).

7. The catheter apparatus of claim 1 wherein the position of the second ultrasound transducer (24b) relative to the first ultrasound transducer (24a) is adjustable.

8. The catheter apparatus of claim 7 comprising an actuatable element (36) coupled to the elongated shaft (16) that, when actuated, is configured to change the position of the second ultrasound transducer (24b) relative to the first ultrasound transducer (24a).

9. The catheter apparatus of claim 7 wherein a length of the elongated shaft (16) between the first ultrasound transducer (24a) and the second ultrasound transducer (24b) is adjustable.

10. The catheter apparatus of claim 1 wherein the first ultrasound transducer (24a) or the second ultrasound transducer (24b) comprises components that are adjustable relative to one another.

11. The catheter apparatus of claim 1 wherein the first ultrasound transducer (24a) or the second ultrasound transducer (24b) comprises a cylindrical or barrel shape.

12. The catheter apparatus of claim 1 wherein the first ultrasound transducer (24a) comprises an annular transducer or an array of transducers that form a ring.

13. The catheter apparatus of claim 1 wherein the second transducer (24b) is capable of being focused in two dimensions.

14. The catheter apparatus of claim 1 wherein the second transducer (24b) is capable of being rotated about an axis of the elongated shaft (16) and/or wherein the second transducer (24b) is capable of being vertically deflected relative to an axis of the elongated shaft (16).

15. The catheter apparatus of claim 1 wherein at least one dimension of the first transducer (24a) is larger than a diameter of the renal artery of the patient.


**Patentansprüche**

1. Kathetervorrichtung für die intravaskuläre Modulation von renalen Nerven, wobei die Kathetervorrichtung Folgendes umfasst:

   einen länglichen Schaft (16) mit einem proximalen Teil (18) und einem distalen Teil (20), wobei der distale Teil (20) des Schafts (16) für die intravaskuläre Übertragung an eine Nierenarterie eines Patienten konfiguriert ist; einen ersten Ultraschallwandler (24a), der mit dem distalen Teil (20) des Schafts (16) verbunden ist und derart konfiguriert ist, dass er innerhalb einer Bauchaorta positioniert werden kann; und einen zweiten Ultraschallwandler (24b), der mit dem distalen Teil (20) des Schafts (16) derart verbunden ist, dass der zweite Ultraschallwandler (24b) um etwa 5 mm bis etwa 10 cm in distaler Richtung von dem ersten Wandler (24a) versetzt ist und wobei der zweite Ultraschallwandler (24b) so konfiguriert ist, dass er innerhalb der Nierenarterie positioniert ist, während der erste Ultraschallwandler (24a) in der Bauchaorta positioniert ist, wobei der erste Ultraschallwandler so konfiguriert ist, dass er innerhalb einer Bauchaorta positioniert ist, wenn entweder durch den ersten Ultraschallwandler (24a) oder den zweiten Ultraschallwandler (24b) oder gleichzeitig durch den ersten Ultraschallwandler (24a) und den zweiten Ultraschallwandler (24b) Energie an die Arterienwand übertragen wird.

2. Kathetervorrichtung nach Anspruch 1, wobei der längliche Schaft (16) ein Lumen umfasst, das so konfiguriert ist, dass es einen Führungsdraht aufnimmt.

3. Kathetervorrichtung nach Anspruch 1, wobei der längliche Schaft (16), der erste Ultraschallwandler (24a) und der zweite Ultraschallwandler (24b) hinsichtlich ihrer Größe und Konfiguration für die intravaskuläre Übertragung über einen 6 Charriere (2 mm) großen oder einen kleineren Führungskatheter ausgelegt sind.

4. Kathetervorrichtung nach Anspruch 1, wobei mindestens einer von dem ersten Ultraschallwandler (24a) oder dem zweiten Ultraschallwandler (24b) einen Bildgebungswandler umfasst.

5. Kathetervorrichtung nach Anspruch 1, wobei der erste Ultraschallwandler (24a) und der zweite Ultraschallwandler (24b) Therapiewandler umfassen.

6. Kathetervorrichtung nach Anspruch 1, ferner umfassend eine Quelle zum Kühlen des Infusionsmittels, das an ein Übertragungslumen in dem länglichen Schaft angeschlossen ist, wobei das Übertragungslumen eine Öffnung umfasst, die an dem distalen Teil (20) des länglichen Schafts (16) angeordnet ist.

**7.** Kathetervorrichtung nach Anspruch 1, wobei die Position des zweiten Ultraschallwandlers (24b) in Bezug auf den ersten Ultraschallwandler (24a) anpassbar ist.

**8.** Kathetervorrichtung nach Anspruch 7, umfassend ein betätigbares Element (36), das an den länglichen Schaft (16) angeschlossen ist, welches, wenn es betätigt wird, so konfiguriert ist, dass es die Position des zweiten Ultraschallwandlers (24b) in Bezug auf den ersten Ultraschallwandler (24a) verändert.

**9.** Kathetervorrichtung nach Anspruch 7, wobei eine Länge des länglichen Schafts (16) zwischen dem ersten Ultraschallwandler (24a) und dem zweiten Ultraschallwandler (24b) anpassbar ist.

**10.** Kathetervorrichtung nach Anspruch 1, wobei der erste Ultraschallwandler (24a) oder der zweite Ultraschallwandler (24b) Komponenten umfasst, die in Bezug auf einander anpassbar sind.

**11.** Kathetervorrichtung nach Anspruch 1, wobei der erste Ultraschallwandler (24a) oder der zweite Ultraschallwandler (24b) eine Zylinder- oder Fassform umfasst.

**12.** Kathetervorrichtung nach Anspruch 1, wobei der erste Ultraschallwandler (24a) einen ringförmigen Wandler oder eine Reihe von Wandlern umfasst, die einen Ring bilden.

**13.** Kathetervorrichtung nach Anspruch 1, wobei der zweite Wandler (24b) in der Lage ist, in zwei Maßen fokussiert zu sein.

**14.** Kathetervorrichtung nach Anspruch 1, wobei der zweite Wandler (24b) in der Lage ist, um eine Achse des länglichen Schafts (16) gedreht zu werden und/oder wobei der zweite Wandler (24b) in der Lage ist, vertikal in Bezug auf eine Achse des länglichen Schafts (16) umgelenkt zu werden.

**15.** Kathetervorrichtung nach Anspruch 1, wobei mindestens ein Maß des ersten Wandlers (24a) größer als ein Durchmesser der renalen Arterie des Patienten ist.


**Revendications**

**1.** Appareil à cathéter pour la modulation intravasculaire des nerfs rénaux, l'appareil à cathéter comprenant :

une tige allongée (16) comportant une partie proximale (18) et une partie distale (20), la partie distale (20) de la tige (16) étant configurée pour l'introduction intravasculaire dans l'artère rénale d'un patient ;
un premier transducteur à ultrasons (24a) associé à la partie distale (20) de la tige (16) et configuré pour être positionné à l'intérieur de l'aorte abdominale ; et
un second transducteur à ultrasons (24b) associé à la partie distale (20) de la tige (16) de telle sorte que le second transducteur à ultrasons (24b) est éloigné d'environ 5 mm à environ 10 cm du premier transducteur (24a) dans une direction distale, et dans lequel le second transducteur à ultrasons (24b) est configuré pour être positionné à l'intérieur de l'artère rénale tandis que le premier transducteur à ultrasons (24a) est positionné dans l'aorte abdominale,
dans lequel le premier transducteur à ultrasons est configuré pour être positionné à l'intérieur de l'aorte abdominale lorsque l'énergie est délivrée à la paroi artérielle soit par le premier transducteur à ultrasons (24a) ou le second transducteur à ultrasons (24b) soit simultanément par le premier transducteur à ultrasons (24a) et le second transducteur à ultrasons (24b).

**2.** Appareil à cathéter selon la revendication 1, dans lequel la tige allongée (16) comprend une lumière configurée pour recevoir un fil guide.

**3.** Appareil à cathéter selon la revendication 1, dans lequel la tige allongée (16), le premier transducteur à ultrasons (24a), et le second transducteur à ultrasons (24b) sont dimensionnés et configurés pour l'introduction intravasculaire par l'intermédiaire d'un cathéter guide de 6 unités Charrière (2 mm) ou plus petit.

4. Appareil à cathéter selon la revendication 1, dans lequel au moins l'un du premier transducteur à ultrasons (24a) ou du second transducteur à ultrasons (24b) constitue un transducteur d'imagerie.

5. Appareil à cathéter selon la revendication 1, dans lequel le premier transducteur à ultrasons (24a) et le second transducteur à ultrasons (24b) constituent des transducteurs de traitement.

6. Appareil à cathéter selon la revendication 1, comprenant en outre une source de perfusat de refroidissement couplé à une lumière d'introduction dans la tige allongée, dans lequel la lumière d'introduction comprend une ouverture localisée dans la partie distale (20) de la tige allongée (16).

7. Appareil à cathéter selon la revendication 1, dans lequel la position du second transducteur à ultrasons (24b) relativement au premier transducteur à ultrasons (24a) est ajustable.

8. Appareil à cathéter selon la revendication 7, comprenant un élément actionnable (36) couplé à la tige allongée (16) qui, lorsque actionné, est configuré pour changer la position du second transducteur à ultrasons (24b) relativement au premier transducteur à ultrasons (24a).

9. Appareil à cathéter selon la revendication 7, dans lequel la longueur de la tige allongée (16) entre le premier transducteur à ultrasons (24a) et le second transducteur à ultrasons (24b) est ajustable.

10. Appareil à cathéter selon la revendication 1, dans lequel le premier transducteur à ultrasons (24a) ou le second transducteur à ultrasons (24b) comprend des composants qui sont ajustables les uns relativement aux autres.

11. Appareil à cathéter selon la revendication 1, dans lequel le premier transducteur à ultrasons (24a) ou le second transducteur à ultrasons (24b) comprend une forme cylindrique ou de tonneau.

12. Appareil à cathéter selon la revendication 1, dans lequel le premier transducteur à ultrasons (24a) constitue un transducteur annulaire ou un réseau de transducteurs qui forment un anneau.

13. Appareil à cathéter selon la revendication 1, dans lequel le second transducteur (24b) est capable d'être focalisé dans deux dimensions.

14. Appareil à cathéter selon la revendication 1, dans lequel le second transducteur (24b) est capable de tourner autour d'un axe de la tige allongée (16) et/ou dans lequel le second transducteur (24b) est capable d'être dévié verticalement relativement à l'axe de la tige allongée (16).

15. Appareil à cathéter selon la revendication 1, dans lequel au moins une dimension du premier transducteur (24a) est plus grande qu'un diamètre de l'artère rénale du patient.

FIG. 1

FIG. 2

27

FIG. 3A

CNS Integration

Smooth Muscle Migration
Vasoconstriction
Atherosclerosis

Hypertrophy
Arrhythmias
Ischemia
Heart Failure

Renal Afferent Nerves

Renal Efferent Nerves

Renal Ischemia
↓ Stroke Volume
↑ Adenosine

Renal Afferent
Nerves

↑ Renis Release
  RAAS
  Systematic Sym Gain
↑ Na+ Retention
  Hypervolemia
  Wall Stiffness
↓ Decreased RBF
↑ Proteinuria
↑ BNP Resistance

FIG. 3B

28

FIG. 4A
*Arterial Vasculature*

FIG. 4B
*Venous Vasculature*

FIG. 5

FIG. 6

FIG. 7A

FIG. 7B

FIG. 7C

74

24

12

20

80

FIG. 7D

82a

24

FIG. 7E

24

82b

74

24

20

84

16

FIG. 7F

FIG. 8

FIG. 9A

FIG. 9B

FIG. 10

FIG. 11

FIG. 12

FIG. 13

24a

24b    182

20

16

24    208

200a    200b

210    206    204

FIG. 14A

FIG. 14B

FIG. 14C

FIG. 14D

FIG. 14E

FIG. 14F

FIG. 14G

FIG. 15

24a

260

FIG. 16

24a

EP 2 635 348 B1

270  24  260  272

FIG. 17

FIG. 18

FIG. 19

**REFERENCES CITED IN THE DESCRIPTION**

**Patent documents cited in the description**

- WO 2008003058 A

- US 545648 A